# EUROPEAN PATENT SPECIFICATION

(11) **EP 3 714 904 B1**
(45) Date of publication and mention of the grant of the patent: **23.08.2023**
(21) Application number: 19386020.2
(22) Date of filing: 27.03.2019
(51) Int. Cl.: A61K 47/40, C08B 37/16, A61P 31/04, A61K 9/00, A61K 9/08, A61K 47/02

(54) **IMINODIACETIC ACID SUBSTITUTED CYCLODEXTRINS AS POTENTIATORS OF BETA-LACTAM ANTIBIOTICS**
IMINODIESSIGSÄURE-SUBSTITUIERTE CYCLODEXTRINE ALS POTENTIATOREN VON BETA-LACTAM-ANTIBIOTIKA
CYCLODEXTRINES SUBSTITUÉES PAR UN ACIDE IMINODIACÉTIQUE EN TANT QUE POTENTIALISATEURS D'ANTIBIOTIQUES BÊTALACTAMIQUES

(43) Date of publication of application: 30.09.2020
(73) Proprietor: NATIONAL CENTER FOR SCIENTIFIC RESEARCH "DEMOKRITOS" (NCSRD), 15341 Agia Paraskevi Attikis (GR); Hellenic Pasteur Institute (HPI), 11521 Athens (GR)
(72) Inventor: Yannakopoulou, Konstantina, 15341 Agia Paraskevi Attikis (GR); Agnes, Marco, 15341 Agia Paraskevi Attikis (GR); Miriagou, Vivi, 11521 Athens (GR); Kotsakis, Stathis-Aristomenis, 11521 Athens (GR); Miliotis, Georgios, 11521 Athens (GR)
(74) Representative: Roukounas, Dimitrios

(56) References cited:
- WO-A1-2018/091668
- GR-B- 1 006 924
- DAVIDE MAFFEO ET AL: "Novel polycarboxylated EDTA-type cyclodextrins as ligands for lanthanide binding: study of their luminescence, relaxivity properties of Gd(iii) complexes, and PM3 theoretical calculations", ORGANIC & BIOMOLECULAR CHEMISTRY, vol. 8, no. 8, 1 January 2010 (2010-01-01) , page 1910, XP055626401, ISSN: 1477-0520, DOI: 10.1039/b924980j
- JING-ZHEN DENG: "Methicillin/per-6-(4-methoxylbenzyl)-amin o-6-deoxy-[beta]-cyclodextrin 1:1 complex and its potentiation in vitro against methicillin-resistant Staphylococcus aureus", THE JOURNAL OF ANTIBIOTICS, vol. 66, no. 9, 29 May 2013 (2013-05-29), pages 517-521, XP055626404, GB ISSN: 0021-8820, DOI: 10.1038/ja.2013.51
- DAVIDE MAFFEO ET AL: "Positive effect of natural and negatively charged cyclodextrins on the stabilization of penicillins towards [beta]-lactamase degradation due to inclusion and external guest-host association. An NMR and MS study", ORGANIC & BIOMOLECULAR CHEMISTRY, vol. 4, no. 7, 1 January 2006 (2006-01-01) , page 1297, XP055626400, ISSN: 1477-0520, DOI: 10.1039/b517275f

## Description

### FIELD OF THE INVENTION

The invention relates to the use of hexakis-, heptakis- and octakis(6-deoxy-6-IDA)cyclodextrins and hexakis-, heptakis- and octakis(6-deoxy-6-IDA-2,3-*O*-methyl)cyclodextrins (IDA = iminodiacetic acid) to potentiate β-lactam antibiotics in the treatment of bacterial infections caused by Gram-negative pathogens producing metallo-β-lactamases (MBLs).

### BACKGROUND OF THE INVENTION

Antibiotic-resistant bacteria are frequently encountered in healthcare settings where antibiotic selective pressure is intensive (C. L. Ventola, The antibiotic resistance crisis: Part 1: Causes and threats, Pharm. Ther. 2015, 40(4), 277 - 283; J. Davies and D. Davies, Origins and evolution of antibiotic resistance, Microbiol. Mol. Biol. Rev. 2010, 74(3), 417 - 433; R. Laxminarayan et al., Antibiotic resistance - The need for global solutions, Lancet Infect. Dis. 2013, 13(12), 1057 - 1098). Moreover, isolation of resistant strains in the community, in food-producing and companion animals and in various environmental sources steadily increases. The problem has been amply recognized by the scientific community and the WHO (report, 15 February 2018, http://www.who.int/news-room/fact-sheets/detail/antimicrobial-resistance) that call for immediate action.

β-Lactams i.e antibiotics containing a β-lactam ring (such as penicillins, cephalosporins, monobactams and carbapenems) are the most frequently used antimicrobial agents due to their wide activity spectra, high bactericidal efficacy, and a nearly optimal selective toxicity. The primary mechanism of β-lactam resistance in Gram-negative pathogens is production of β-lactamases i.e. enzymes hydrolyzing the amide bond of the β-lactam ring.

Carbapenems (G. G. Zhanel, R. Wiebe, L. Dilay, K. Thomson, E. Rubinstein, D. J. Hoban, A. M. Noreddin, and J. A. Karlowsky, Comparative review of the carbapenems, Drugs 2007, 67(7), 1027 - 1052) are among the most reliable last line antibiotics for the treatment of life-threatening infections. This has led to their increased use facilitating the emergence of carbapenem-resistant *Enterobacteriaceae* (CRE) (mainly *Klebsiella pneumoniae* and less frequently *Escherichia coli* and other enterobacterial species) (N. Gupta, B. M. Limbago, J. B. Patel, A. J. Kallen, Carbapenem-Resistant Enterobacteriaceae: Epidemiology and Prevention, Clin. Infect. Diseases, 2011, 53(1), 60 -67).

CRE phenotype is conferred by a variety of acquired β-lactamases, the most clinically important being so far the KPC, OXA, NDM, VIM, and IMP types, collectively called "carbapenemases". Carbapenemases are distributed into three of the four molecular classes of β-lactamases (A, B, C, and D) (A. M. Queenan, K. Bush, Carbapenemases: the versatile β-lactamases, Clinical Microbiol. Rev. 2007, 20(3), 440 - 458). KPC and OXA derivatives belong to classes A and D, respectively. Hydrolysis of the amide bond of the β-lactam ring is mediated by a conserved serine residue present in the active site (serine β-lactamases) (L. S. Tzouvelekis, R. A. Bonomo, SHV-type beta-lactamases, Current Pharm. Design 1999, 5(11), 847 - 864; B. Jaurin, T. Grundstrom, ampC Cephalosporinase of Escherichia coli K-12 has a different evolutionary origin from that of β-lactamases of the penicillinase type, Proc. Natl. Acad. Sci. USA, 1981, 78(8), 4897 - 4901). NDM, VIM, and IMP β-lactamases, classified into class B enzymes, are characterized by active sites containing two divalent cations (Zn⁺²) required for β-lactam hydrolysis through activation of a nucleophilic water molecule (metallo-β-lactamases [MBLs]) (T. R. Walsh, M. A. Toleman, L. Poirel, P. Nordmann, Metallo-β-lactamases: the quiet before the storm? Clin. Microbiol. Rev., 2005, 18(2), 306 - 325).

Multidrug- and carbapenem-resistant enterobacterial strains, in particular those producing KPC- and NDM-type β-lactamases, have achieved global spread being epidemic in various geographic areas [L. S. Tzouvelekis, A. Markogiannakis, M. Psichogiou, P. T. Tassios, G. L. Daikos, Carbapenemases in Klebsiella pneumoniae and other Enterobacteriaceae: an evolving crisis of global dimensions, Clin Microbiol Rev. 2012, 25(4), 682 - 707]. Given the limited therapeutic options, unacceptably high fatality rates (>40%) among CRE infected patients have been reported (L. Chen, R. Todd, J. Kiehlbauch, M. Walters, A. Kallen, Notes from the Field: Pan-resistant New Delhi Metallo-beta-lactamase-producing Klebsiella pneumoniae - Washoe County, Nevada, 2016. Morb. Mortal. Wkly. Rep. 2017, 66:33).

For the last 25 years no new classes of antibiotics have been introduced in the clinic against Gram-negative bacteria. With antimicrobial resistance swiftly rising, there has been a need to invent combinations of the currently available antibiotics with other compounds to avoid the consequences of panresistant bacterial pandemics. The successful example of the co-administration of amoxicillin with the β-lactamase inhibitor clavulanic acid to overcome antibiotic resistance due to β-lactamases paves the way for similar combinations to be developed.

Intensive research efforts have been directed towards the invention of compounds active against carbapenemase producers. Against CRE, significant progress has been made in the field of novel inhibitors such as avibactam, relebactam and vaborbactam. The latter compounds in combination with ceftazidime and meropenem respectively, are already in clinical use. These brand new β-lactamase inhibitor types exhibit wide inhibitory spectra against most class A, C, and D bacterial enzymes including serine carbapenemases. Yet, they are inactive against MBLs (class B).

Development of MBL inhibitors for clinical use has been proven difficult. Indeed, of the numerous MBL inhibitors described thus far [U. I. Faridoon, N. Islam, An Update on the Status of Potent Inhibitors of Metallo-β-Lactamases, Scientia Pharmaceutica, 2013; 81(2), 309 - 327] none has yet been introduced in therapeutics. Expectedly, most of these compounds act either as binders or chelators "targeting" the active site's zinc cations. Unwarranted toxicity due to inhibition of mammalian metallo-enzymes is likely the main obstacle in producing inhibitors suitable for the treatment of infections caused by MBL producing CRE. Consequently, effective and safe compounds, of suitable pharmacokinetic and biodistribution profile that can be readily prepared in a scalable manner and that can selectively inhibit the activity of MBLs are urgently needed especially for treatment of life threatening infections such as sepsis.

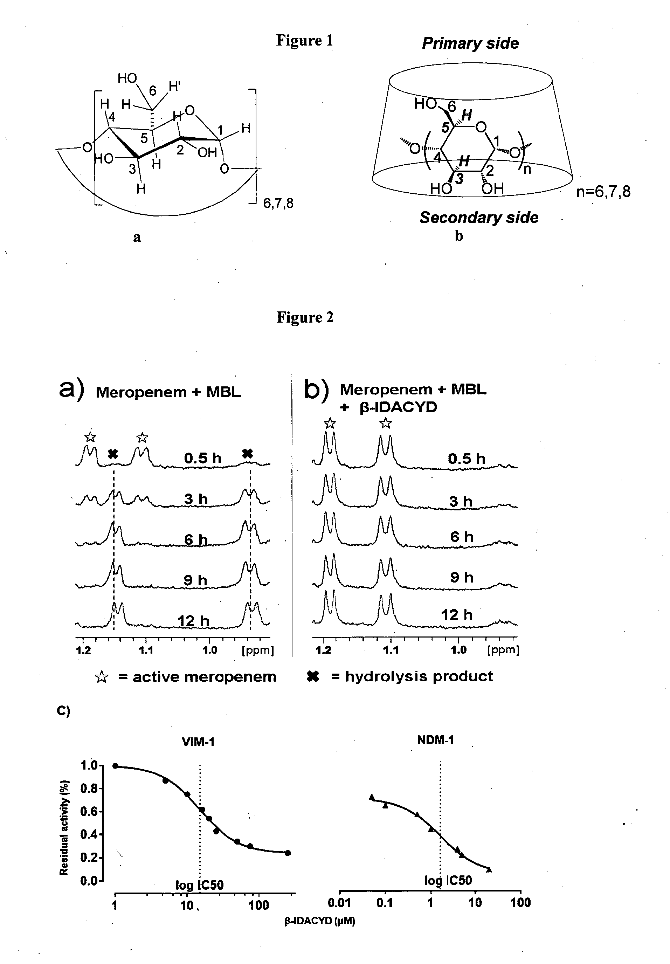

Cyclodextrins (CDs) are cyclic oligosaccharides formed by 6, 7 and 8 α-D-glucopyranose units (as in Figure 1a) connected via 1,4-bonds in a circle (α-, β- and γ-cyclodextrins, respectively). CDs form a cavity and they are generally schematically depicted as truncated cones as shown in Figure 1b. The CDs are structurally characterized by hydrophilic exterior (-CH₂*OH,* at the primary side and -CH*OH* at the secondary side of the cone) and a lipophilic internal cavity, that can encapsulate lipophilic molecules insoluble in water and thus form inclusion or host-guest complexes (J. Szejtli, Introduction and general overview of cyclodextrin chemistry, Chem. Rev. 1998, 98, 1743 - 1753).

Synthetically modified cyclodextrins or cyclodextrin derivatives refer to cyclodextrin compounds, in which the hydroxyl groups in carbon atoms C6 (of the primary side) or/and C2 or/and C3 (of the secondary side) have been substituted by one or more atoms or groups of atoms.

The cyclodextrin derivatives generally referred as IDACYDs and specifically *hexakis-, heptakis-* and *octakis* (6-deoxy-6-IDA-2,3-*O*-methyl) cyclodextrins (IDA = iminodiacetic acid) are capable of forming complexes with lanthanide metal cations [D. Maffeo, M. Lampropoulou, M. Fardis, Y. G. Lazarou, I. M. Mavridis, D. A.I. Mavridou, E. Urso, H. Pratsinis, D. Kletsas, K. Yannakopoulou, Novel polycarboxylated cyclodextrins as ligands for lanthanide binding: study of their luminescence, relaxivity properties of Gd(III) complexes, and PM3 theoretical calculations, Org. Biomol. Chem., 2010, 8, 1910 - 1921]. These compounds and their applications for imaging have been disclosed in the Greek patent application 1006924.

Jing-Zhen Deng: " Methicillin/per-6-(4-methoxybenzyl)amino-6-deoxy-[beta]-cyclodextrin 11: 1 complex and its potentiation in vitro against methicillin -resistant Staphylococcus aureus" J. Antibiotics., vol. 66, no. 9., pp.217 (2013) discloses a β-cyclodextrin derivative and its use against two methicillin-resistant Staphylococcus aureus strains.

Davide Maffeo ET. AL: "Positive effect of natural and negatively charged cyclodextrins on the stabilization of penicillins towards [beta]-lactamase degradation due to inclusión and external guest-host association. An NMR and MS study" Org. Biomol. Chem., vol. 4, no. 7, pp.1297 (2006) discloses cyclodextrin derivatives and their inclusion complexes with penicillines.

### SUMMARY OF THE INVENTION

The present invention addresses the serious limitations of existing treatments for severe infections.

The present invention provides **IDACYD**s as a new class of potent inhibitors of bacterial MBLs: Furthermore, it provides iminodiacetic acid-substituted type cyclodextrins (**IDACYD**s) in combination with β-lactam antibiotics for use in the treatment of bacterial infections caused by Gram-negative pathogens producing MBLs.

The **IDACYD**s of the present invention exhibit potentiation and/or reactivation of β-lactam antibiotics. This enables the treatment with β-lactam antibiotics of bacterial infections not treatable with the currently available antibiotics and antibiotic combinations. On the other hand, the **IDACYD**s of the present invention exhibit no cytotoxicity, no acute toxicity and they do not induce immune or inflammatory response.

The present invention also provides pharmaceutical compositions comprising **IDACYD**s for use in the treatment of bacterial infections caused by Gram-negative pathogens producing MBLs.

### BRIEF DESCRIPTION OF DRAWINGS

**Fig. 1** shows the glucopyranose units of CDs and the cavity they form.
**Fig. 2** shows the ¹H NMR spectra of meropenem in the presence of an MBL containing crude enzyme preparation monitored over time showing a) hydrolysis of meropenem in the absence and b) protection in the presence of **β-IDACYD**, while c) is the determination of IC₅₀ against two MBLs.
**Fig. 3** shows the time-kill assays of *K. pneumoniae* strains using meropenem and imipenem at inhibitory concentrations and demonstrates that **IDACYD**s completely prevent bacterial population regrowth overtime.
**Fig. 4** shows the lack of cytotoxicity of **β-IDACYD** in *in-vitro* assays.
**Fig. 5** shows the stability and bioavailability in human and mouse plasma and the biodistribution profile in mice using radiolabeled **⁶⁷Ga-β-IDACYD**.
**Fig. 6** shows the bacterial load of NDM-1 producing *K. pneumoniae* strain and survival percentage of mice treated with a 2-hour intervals treatment scheme of meropenem in the absence or presence of **β-IDACYD** in an *in-vivo* murine sepsis model.
**Fig. 7** shows the bacterial load of NDM-1 producing *K. pneumoniae* strain and survival percentage of mice treated with two single dose regimens of meropenem in the absence or presence of **β-IDACYD** in an *in-vivo* murine sepsis model.
**Fig. 8** shows that the plasma concentration of calcium, iron, phosphate, magnesium ions as well as urea and creatine derived from infected mice under meropenem treatment is not affected by the presence of **β-IDACYD.**

### DETAILED DESCRIPTION OF THE INVENTION

The present invention provides a compound according to Formula I 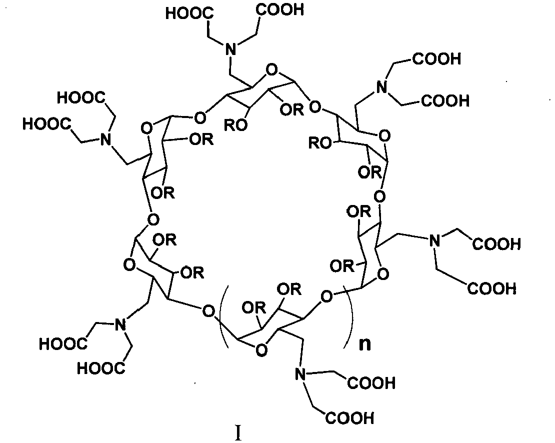 wherein R is CH₃ or H, and n is 1, 2 or 3, for use in the treatment of a bacterial infection caused by a Gram-negative bacterium producing a metallo-β-lactamase, wherein the compound is administered in combination with a β-lactam antimicrobial agent. Preferably, R is CH₃ and n is 2. Compounds according to formula I wherein R is H and n is 1, 2 or 3 are new.

The compounds of Formula I are *hexakis-, heptakis-* or *octakis*(6-deoxy-6-IDA)cyclodextrins (IDA = iminodiacetic acid) and are generally referred to in the present application as **IDACYDs.** Specifically, the compounds of Formula I wherein R is CH₃ and n = 1, 2 or 3 are **α-IDACYD, β-IDACYD** and **γ-IDACYD,** respectively. The compounds of Formula I wherein R is H and n = 1, 2 or 3 are **α-IDACYD-OH, β-IDACYD-OH** and **γ-IDACYD-OH,** respectively.

β-Lactams (such as penicillins, cephalosporins, monobactams and carbapenems) are the most frequently used antimicrobial agents due to their wide activity spectra, high bactericidal efficacy, and a nearly optimal selective toxicity. Additionally, modifications of the side chains attached to the β-lactam nuclei allow development of compounds with distinct pharmacokinetics. The primary mechanism of β-lactam resistance in Gram-negatives is production of β-lactamases i.e. enzymes hydrolyzing the amide bond of the β-lactam ring resulting in inactivation of the antibiotic. The development of β-lactamase inhibitors is one of the strategies for the treatment of infections caused by β-lactamase producing bacteria.

Devising MBL inhibitors for clinical use has been proven difficult. Indeed, of the numerous MBL inhibitors described thus far none has yet been introduced in therapeutics.

It has now surprisingly been found that a compound according to Formula I inactivates metallo-β-lactamases and therefore potentiates clinically available β-lactams against MBL-producing bacteria.

An **IDACYD** of the present invention can be used in combination with a β-lactam antimicrobial agent. Examples of β-lactam antimicrobial agents are shown in Table 1 below.

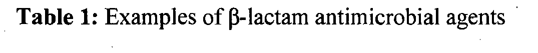

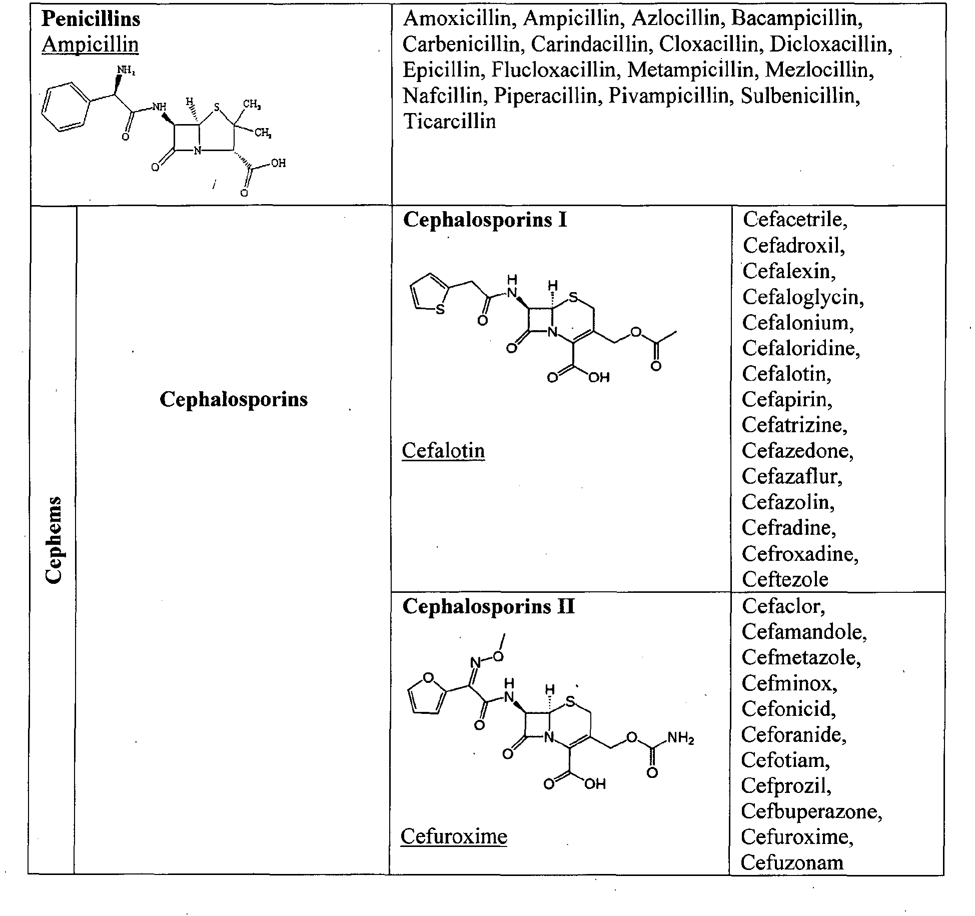

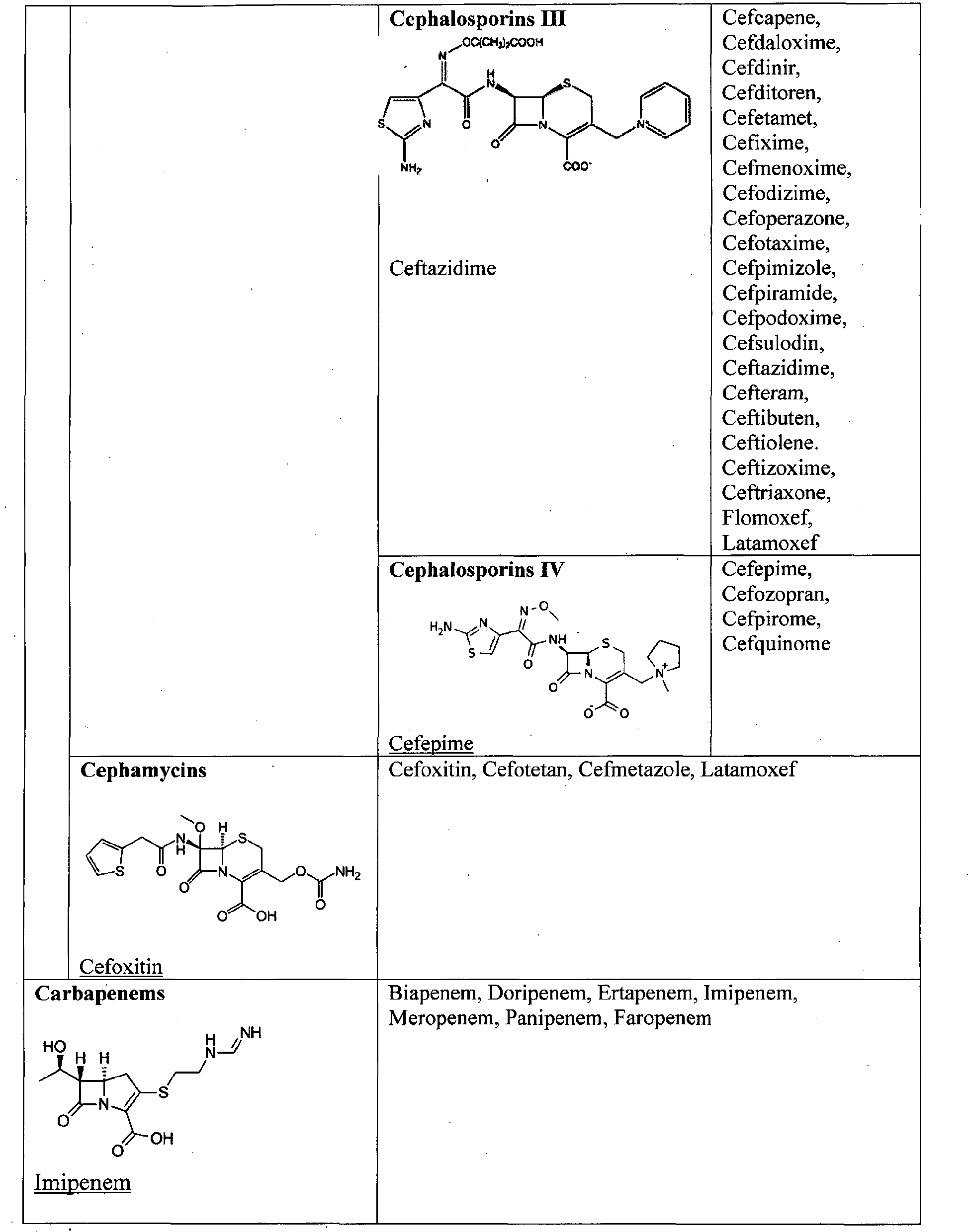

Preferably, the β-lactam antimicrobial agent according to the invention is a cephalosporin or a carbapenem. More preferably, the P-lactam antimicrobial agent is a carbapenem.

A compound according to the present invention can be used for the treatment of a bacterial infection in a human or animal subject. Preferably, the subject is a human.

The combinations of **IDACYD**s of the present invention with β-lactams can be used for the treatment of Health Care Associated (HA) and Community Acquired (CA) infections caused by MBL-producing bacteria (Enterobacteriaceae and non-fermenting bacilli). Examples of diseases arising from MBL-producing bacterial infections are listed herein below. The list of infections and also the list of MBL-producing bacterial species are not exhaustive, due to the genetic background of MBLs. Indeed, MBL genes reside on a wide variety of mobile structures facilitating their dissemination in numerous bacterial species. Bacterial infections caused by enterobacteria (mainly *K. pneumoniae* and *E.coli*) and non-fermenting species (*Pseudomonas aeruginosa* and *Acinetobacter* spp.) are, for example, but not limited to, blood stream infections-bacteraemia (primary and secondary), septicemia, sepsis, septic shock, upper respiratory tract infections, lower respiratory tract infections (bronchopneumonia or bronchitis), lung abscesses, urinary tract infections (uncomplicated and complicated including pyelonephritis), intra-abdominal infections (cholecystitis, diarrhea, lower biliary tract infections), surgical wound infections, endocarditis, skin and soft tissue infections, thrombophlevitis, osteomyelitis, septic arthritis, endophthalmitis, cystic fibrosis lung infection, brain abscesses, infections due to the use of medical devices and hospitalization, or a combination thereof.

An **IDACYD** of the present invention is administered in combination with a β-lactam antimicrobial agent. The term "combination" includes simultaneous and successive administration of the **IDACYD** and the β-lactam. In the case of simultaneous administration, the **IDACYD** and the β-lactam may be present in the same or separate pharmaceutical compositions. In the case of successive administration, the **IDACYD** can be administered before or after the β-lactam. In these cases the second ingredient is preferably administered within 15-30 minutes of the administration of the first ingredient. More preferably, the second ingredient is administered within 20 minutes of the administration of the first ingredient.

An **IDACYD** of the present invention is preferably administrated at a dosage of 5 to 500 mg/kg. More preferably, the dosage ranges from 10 to 120 mg/kg. The dosage and the time interval between dosages can be easily determined by a person skilled in the art in order to achieve the maximum desired result which reduces the bacterial load, significantly ameliorates disease symptoms arising from them and improves the antibiotic's therapeutic efficiency.

The ratio between the administered amount of a β-lactam antimicrobial agent and an **IDACYD** according to the present invention preferably ranges from 1/0.3 to 1/15. More preferably, the ratio ranges from 1/0.7 to 1/12.The amount of the administrated compounds can vary depending on several factors such as site of infection and route of administration and can be easily determined by a person skilled in the art.

An **IDACYD** of the present invention can be administered via different routes such as oral or parenteral (including intravenous and intramuscular). Preferably, the **IDACYD** is administered via the parenteral route. The pharmacokinetic parameters for intravenously administered **IDACYDs** are in line with the respective pharmacokinetic parameters for β-lactam antibiotics, which is a major advantage for achieving the maximum efficacy of the therapeutic combinational schemes.

The **IDACYD**s of the present invention exhibit no cytotoxicity, no acute toxicity and they do not induce immune or inflammatory response.

The present invention also provides a pharmaceutical composition comprising an **IDACYD** of the present invention. Such a pharmaceutical composition may be formulated for administration by any appropriate route such as oral, topical or parenteral route. For example, a pharmaceutical composition may be formulated as tablet, capsule, powder, solution or suspension. Such a composition generally contains, in addition to an **IDACYD**, a pharmaceutically acceptable carrier. Such a carrier comprises excipients well known in the art, such as diluents, binders, fillers, disintegrants, lubricants, solvents, suspending agents, thickening agents, buffers, and preservatives. The composition may be prepared following methods well known in the art.

A pharmaceutical composition according to the invention may in addition to an **IDACYD** comprise a β-lactam antimicrobial agent. Alternatively, the β-lactam antimicrobial agent may be formulated in a separate pharmaceutical composition. In the latter case, the two compositions may be combined prior to the administration, if possible.

### EXAMPLES

### Example 1. Synthesis of IDACYD compounds according to Formula I where R is CH₃ and n is 1 (a-IDACYD), R is CH₃ and n is 2 (β-IDACYD), R is CH₃ and n is 3 (γ-IDACYD).

The synthesis described herein comprises two steps, starting from the literature compounds hexakis(6-amino-6-deoxy-2,3-di-*O*-methyl)-aCD and heptakis(6-amino-6-deoxy-2,3-di-*O*-methyl)-βCD (D. A. Fulton, A. R. Pease and J. F. Stoddart, Cyclodextrin-based clusters by amide bond formation, Isr. J. Chem., 2000, 40, 325-333.) and octakis(6-amino-6-deoxy-2,3-di-*O*-methyl)-yCD (T. Kraus, M. Budesinsky and J. Zavada, Synthesis of per-6-guanidinylated cyclodextrins, Tetrahedron Lett., 2006, 47, 679-681.):
The hydrochloride salt of per(6-amino-6-deoxy-2,3-di-*O*-methyl)-α-, -β- and -γCDs(α-: 0.374 g, 0.28 mmol; β-: 0.151 g, 0.10 mmol; γ-: 0.397 g, 0.22 mmol) was dispersed in dry acetonitrile (10 / 7 / 15 mL) and t-butyl bromoacetate (5.5 / 1.5 / 1.7 mL, 30.2 / 10.2 / 11.5 mmol), K₂CO₃ (1.102 / 0.508 / 1.766 g, 7.97 / 3.67 / 12.8 mmol) and potassium iodide (0.048 / 0.153 / 0.034 g, 0.29 / 0.92 / 0.20 mmol) were added in different portions over the reaction time (3 - 6 days). The mixture was stirred under Argon atmosphere at reflux (80 °C) and the reaction was monitored by thin layer chromatography (CH₂Cl₂: methanol, 95 : 5, v/v. R_{f-Start} = 0.0, R_{f-prod} = 0.5). The reaction mixture was then dried under reduced pressure and the obtained solid was dispersed in water and extracted with CH₂Cl₂. The organic layers were dried over MgSO₄ (1 h) and after filtration the solvents were removed under reduced pressure to give the crude solid. Purification by column chromatography on silica gel (elution in CH₂Cl₂ with increasing amounts of methanol, up to 5%) yielded the desired per[6-bis(*t-*butyloxycarbonylmethyl)amino-6-deoxy-2,3-di-*O*-methyl]-α- / -β- / -γ CDs as pale yellow solids (yields: 82 / 60 / 59 %, respectively):
*Hexakis(6-bis(t-butyloxycarbonylmethyl)amino-6-deoxy-2,3-di-O-methyl]-αCD*: ¹H NMR (500 MHz, CDCl₃, 298 K) δ 5.05 (s, 6H; H1), 4.20 (t, 6H; H4), 3.60 (s, 18H; OMe3), 3.58-3.49 (m, 24H; H5, H3, H7a), 3.46 (s, 18H; OMe2), 3.38 (m, 12H; H7b), 3.25-3.14 (m, 12H; H6a,b), 3.11 (dd, 6H; H2), 1.47 (s, 54H; *t*-Bu); ¹³C NMR (63 MHz, CDCl3) δ 171.2 (C8), 99.6 (C1), 82.2 (C2), 81.7 (C3), 81.0 (C4), 80.2 (C9), 73.5 (C5), 61.8 (Me3), 58.3 (C7), 57.4 (Me2), 55.3 (C6), 28.3 (*t-*Bu). MS (MALDI-TOF) *m*/*z* 2526.4 ([*M*+Na]⁺, calcd: 2526.41). Elemental analysis calculated for C₁₂₀H₂₁₀O₄₈N₆.3H₂O: C, 56.3; H, 8.5; N, 3.3%. Found: C, 55.9; H, 8.3; N 3.5%.

*Heptakis[6-bis(t-butyloxy-carbonylmethyl)amino-6-deoxy-2,3-di-O-methyl]-βCD*: ¹H NMR (500 MHz, CDCl₃, 298 K) δ 5.21 (s, 7H; H1), 4.02 (t, 7H; H4), 3.72 (m, 7H; H5), 3.60 (s, 21H; OMe3), 3.55 (m, 14H; H7a), 3.50 (s, 21H; OMe2), 3.43-3.40 (m, 21H; H3, H7b), 3.23 (br d, 7H; H6a), 3.12-3.07 (br, 14H; H2, H6b), 1.47 (s, 63H; t-Bu); ¹³C NMR (125 MHz, CDCl3) δ 170.9 (C8), 98.9 (C1), 81.9 (C2), 81.4 (C3), 80.9 (C4), 80.3 (C9), 73.1 (C5), 61.3 (Me3), 58.2 (Me2), 57.8 (C7), 55.1 (C6), 28.3 (t-Bu). MS (ESI-TOF) *m*/*z* 1484.3 ([*M*+2Na]²⁺, calcd:1484.1). Elemental analysis calculated for C₁₄₀H₂₄₅O₅₆N₇: C, 57.5; H, 8.45; N, 3.4%. Found: C, 57.8; H, 8.5; N, 3.05%.

*Octakis[6-bis(t-butyloxycarbonylmethyl)amino-6-deoxy-2,3-di-O-methyl]-γCD*: ¹H NMR (500 MHz, CDCl₃, 298 K) δ 5.34 (s, 8H; H1), 4.00 (tr, 8H; H4), 3.64 (m, 8H; H5), 3.60 (s, 24H; OMe3), 3.57-3.52 (m, 16H; H7a), 3.49 (s, 24H; OMe2), 3.46-3.35 (m, 24H; H3, H7b), 3.21 (br d, 8H; H6a), 3.08-3.03 (br m, 24H; H2, H6b), 1.41 (s, 72H; *t*-Bu); 13C NMR (63 MHz, CDCl3) δ 170.9 (C8), 98.2 (C1), 81.8 (C2, C3), 80.1 (C9), 79.7 (C4), 72.8 (C5), 61.5 (Me-3), 58.5 (Me-2), 57.3 (C7), 54.9 (C6), 28.2 (*t*-Bu). MS (MALDI-TOF) *m*/*z* 3361.9 ([*M*+H+Na]⁺, 100%, calculated for C₁₆₀H₂₈₀N₈O₆₄NaH: 3361.89.). Elemental analysis calculated for C₁₆₀H₂₈₀N₈O₆₄.3H₂O: C, 56:6; H, 8.5; N, 3.3%. Found: C, 56.4; H, 8.5; N, 3.3%.

The above compounds were used for the next step to obtain **IDACYDs.**

Per[6-bis(*t*-butyloxycarbonylmethyl)amino-6-deoxy-2,3-di-*O*-methyl]-α-, -β- and -γCDs: α-: 0.565 g, 0.23 mmol; β-: 0.062 g, 0.02 mmol; γ-: 0.437 g, 0.13 mmol) were dissolved in CHCl₃ (≤ 5 mL) and stirred under Argon atmosphere at 40 °C. Trifluoroacetic acid (2 / 0.75 / 2.5 mL, 26.12 / 9.79 / 32.65 mmol) was added to the stirred solutions in different portions over 2 - 3 days, monitoring the reaction by thin layer chromatography (CH₂Cl₂ : methanol, 95 : 5, v/v. Rf_{-Start} = 0.5, R_{f-prod} = 0.0). The solvent CHCl₃ was evaporating during this time and the mixtures were left to stir in the trifluoroacetic acid excess. The reactions were quenched with water and dried under reduced pressure. The solids were collected in water, neutralized using aqueous NaOH (1 M) and the solutions were dialyzed against deionized water for 2 to 5 days. Water was removed under *vacuum* to yield the desired compounds as brownish solids (respective yields: 78 / 85 / 52 %).

**α-IDACYD:** ¹H NMR (500 MHz, D₂O, pH 5, 298 K) δ 5.33 (s, 6H; H1), 4.35 (br, 6H; H5), 3.94 (m, 6H; H3), 3.87 (s, 24H; H7a, b), 3.78 (m, 6H; H4), 3.71 (m, 6H; H6a), 3.55 (s, 18H; OMe3), 3.50 (s, 18H; OMe2), 3.48 (m, 6H; H6b), 3.44 (br m, 6H; H2); ¹³C NMR (125 MHz, D₂O, pH 5, 298 K) δ 170.5 (C8), 97.3 (C1), 79.3 (C4, C3), 78.8 (C2), 68.4 (C5), 59.6 (C7), 59.1 (Me3), 58.0 (Me2), 56.2 (C6). MS (MALDI-TOF) *m*/*z*: 1853.7 ([*M*+Na]⁺, calcd for C₇₂H₁₁₄N₆O₄₈Na: 1853.66). Elemental analysis calculated for C₁₄₀H₂₄₅O₅₆N₇ (%):C 57.54, H 8.45, N 3.36. Found C 57.76, H 8.46, N 3.00%.

**β-IDACYD**: ¹H NMR (500 MHz, D₂O, pH 5, 298 K) δ 5.31 (s, 7H; H1), 4.27 (br, 7H; H5), 3.85 (s, 28H; H7a, b), 3.81-3.75 (m, 14H; H3, H4), 3.65-3.55 (br m, 14H; H6a,b), 3.51 (s, 21H; OMe3), 3.47 (s, 21H; OMe2), 3.43 (br m, 7H; H2); ¹³C NMR (125 MHz, D₂O, pH 5, 298 K) δ 170.4 (C8), 97.0 (C1), 79.4 (C4), 79.0 (C3), 78.2 (C2), 68.1 (C5), 59.2 (C7), 58.8 (Me3), 58.4 (Me2), 55.9 (C6). MS (MALDI-TOF) *m*/*z*: 2158.8 ([*M*+Na]⁺, calcd for C₈₄H₁₃₂N₇O₅₆Na: 2158.99). Elemental analysis calcd. for C₈₄H₁₁₉O₅₆Na₁₄N₇: C 41.27, H 4.91, N 4.01%. Found C 41.16, H 5.38, N 3.92%.

**γ-IDACYD**: ¹H NMR (500 MHz, D₂O, pH 5, 298 K) δ 5.30 (s, 8H; H1), 4.24 (br, 8H; H5), 3.85 (s, 32H; H7a, b), 3.81-3.74 (m, 16H; H3, H4), 3.66 (br m, 8H; H6a), 3.58-3.53 (m, 8H; H6b), 3.52 (s, 24H; OMe3), 3.48 (s, 24H; OMe2), 3.43 (br, 8H; H2); ¹³C NMR (125 MHz, D2O, pH 5, 298 K) δ 170.4 (C8), 96.8 (C1), 79.6 (C4), 79.3 (C3), 77.8 (C2), 67.8 (C5), 59.3 (Me3), 58.7 (C7), 58.5 (Me2), 55.9 (C6). MS (MALDI-TOF) m/z 2463.8 ([*M*+Na]⁺, 100%), 2486.8 ([*M*+2Na]⁺, 66%), calcd for C₉₆H₁₅₂N₈O₆₄ Na: 2463.89. Elemental analysis calculated for C₉₆H₁₃₈N_{g}O₆₄Na_{14.}6H₂O: C, 40.3; H 5.3; N, 3.9%). Found: C, 40.5; H, 5.8; N, 3.8%.

### Example 2. Synthesis of the compound according to Formula I where R is H and n is 2 (β-IDACYD-OH).

The synthesis described herein comprises four steps, starting from the literature compound heptakis(6-azido-6-deoxy)-βCD (N. Mourtzis, K. Eliadou, C. Aggelidou, V. Sophianopoulou, I. M. Mavridis and K. Yannakopoulou, Per(6-guanidino-6-deoxy) cyclodextrins: synthesis, characterisation and binding behaviour toward selected small molecules and DNA, Org. Biomol. Chem., 2007, 5, 125-131).

*Heptakis(6-azido-6-deoxy-2,3-di-O-allyl)-βCD*: A stirred suspension of NaH (0.46 g, 18.2 mmol) in dry dimethyl formamide (15 mL) was cooled down to 0 °C in an ice bath under Argon atmosphere. Heptakis(6-azido-6-deoxy)-βCD (0.5 g, 0.382 mmol) was added all at once to the stirred reaction mixture and diluted with dry dimethyl formamide (15 mL). After 15 min of strong stirring at 0 °C, the flask was transferred into an ultrasound bath for further 15 min, keeping the Argon atmosphere. Dry dimethyl formamide (5 mL) was then added and the mixture was left under intense stirring for further 30 min at 0 °C. Allyl bromide (3 mL, 34.7 mmol) was added drop-wise to the suspension and the mixture became clear. After 10 min of sonication, the reaction was left at 35 °C, with intense stirring and Argon atmosphere for 2 h. After thin layer chromatography monitoring (ethyl acetate: *n*-hexane, 2 : 8, v/v. R_{f-Start} = 0, R_{f-Prod} = 0.5), the reaction was quenched with methanol (20 mL) and the final clear solution was dried under *vacuum.* The residue was collected in brine : H₂O (2 : 1, v/v, 15 mL) and washed with diethyl ether (5 × 10 mL); all the organic layers were combined, dried on MgSO₄ (45 min), filtered and dried under *vacuum.* The desired compound was collected as pale yellow oil (0.709 g, 0.38 mmol, 99%). ¹H NMR (500 MHz, CDCl₃, 298 K): δ 5.93 (m, 7H; H8), 5.85 (m, 7H; H11), 5.23 (d, 7H; H9a), 5.16 (d, 7H; H12a), 5.1 (d, 7H; H9b), 5.04 (d, 7H; H12b), 5.03 (s, 7H; H1), 4.4 (dd, 7H; H8a), 4.19 (dd, 7H; H8b), 4.11 (ddd, 14H; H10), 3.74 (br, 7H; H5), 3.68 (d, 7H; H6a), 3.63 (tr, 7H; H3), 3.55 (m, 14H; H4, H6b), 3.3 (dd, 7H; H2). ¹³C NMR (125 MHz, CDCl₃, 298 K): δ 135.8 (C8), 135.0 (C11), 117.2 (C9), 116.0 (C12), 98.7 (C1), 80.2 (C4), 79.5 (C3), 78.8 (C2), 74.5 (C7), 72.5 (C10), 71.1 (C5), 51.9 (C6). FT-IR: 2100 cm⁻¹ (-N₃ stretching). During MALDI-TOF MS analysis the compound degraded.

*Heptakis(6-amino-6-deoxy-2,3-di-O-allyl)-βCD:* Freshly prepared heptakis(6-azido-6-deoxy-2,3-di-*O*-allyl)-βCD (0.772 g, 0.413 mmol) was dissolved in dimethyl formamide (15 mL) at 35 °C to obtain a clear yellowish solution. Triphenylphosphine (1.108 g, 4.22 mmol) was added to the stirring mixture in three portions over 90 min and the whole was left to stir (750 rpm) at 35 °C under Argon atmosphere for 1 h. After that, NH₃ (25%, aq. - 5 mL) was added drop-wise together with more dimethyl formamide (15 mL) in small portions over 30 min. The reaction mixture was left to stir (750 rpm) at 35 °C under Argon atmosphere for further 16 h. The reaction was monitored by thin layer chromatography (1,4-dioxane : NH₃ (25%, aq.) : *i*-propanol, 10 : 4 : 3 v/ v/v. R_{f-Start} = 0.9, R_{f-Prod} = 0.6). The solution was dried under *vacuum* and the crude residue was collected in brine: NH₃ (25%, aq.) : H₂O (10 : 10 : 10, v/v/v, 30 mL, pH ~ 11) and washed with CH₂Cl₂ (3 × 30 mL). The organic layers were collected together, dried on MgSO₄ (1 h), filtered and dried under *vacuum.* Purification from the triphenylphosphine oxide residues was achieved by column chromatography on silica gel (gradient elution in 1,4-dioxane : NH₃ (25%, aq.) : *i-*propanol, from 10 : 1 : 3 to 10 : 4 : 3, v/v/v). The oily residue was re-dissolved in brine : NH₃ (25%, aq.) : H₂O (5 : 5 : 10, v/v/v, 20 mL, pH ~ 11) and washed with diisopropyl ether (4 × 20 mL). The organic layers were combined, dried on MgSO₄ (1 h), filtered and dried under *vacuum.* The desired compound was collected as a white solid (0.629 g, 0.37 mmol, 90%). ¹H NMR (500 MHz, CDCl₃, 298 K): δ 6.01 (m, 7H; H8), 5.92 (m, 7H; H11), 5.29 (d, 7H; H9a), 5.24 (d, 7H; H12a), 5.15 (d, 7H; H9b), 5.1 (m, 14H; H12b, H1), 4.48 (dd, 7H; H7a), 4.28 (dd, 7H; H7b), 4.17 (m, 14H; H10), 3.74 (br, 7H; H5), 3.72 (d, 7H; H3), 3.56 (t, 7H; H4), 3.32 (dd, 7H; H2), 3.2 (d, 7H; H6a), 2.91 (dd, 7H; H6b). ¹³C NMR (125 MHz, CDCl₃, 298 K): δ 136.2 (C8), 135.3 (C11), 116.9 (C9), 115.8 (C23), 98.4 (C1), 80.2 (C4), 79.9 (C3), 79.3 (C2), 74.4 (C7), 72.6 (C10), 72.3 (C5), 42.8 (C6). FT-IR: 3365 cm⁻¹; 3298 cm⁻¹ (-NH₂ asymmetric stretching). Calc. mass [C₈₄H₁₄₀N₇O₂₈] = 1688; MALDI-TOF MS, m/z: 1689 [M + H]⁺.

*Heptakis[6-bis(t-butyloxycarbonylmethyl)amino-6-deoxy-2,3-di-O-allyl)-βCD: t*-Butyl bromoacetate (3.25 mL, 22 mmol), K₂CO₃ (1.714 g, 12.4 mmol) and potassium iodide (0.127 g, 0.76 mmol) were added in different portions, over a few days, to a solution of heptakis(6-amino-6-deoxy-2,3-di-*O*-allyl)-βCD (0.507 g, 0.3 mmol) in acetonitrile (volume kept constant, about 10 mL). The heterogeneous milky mixture was heated under reflux (bath temperature 80 °C) and vigorously stirred for 4 days. The reaction was monitored by thin layer chromatography (CH₂Cl₂ : methanol, 99 : 1, v/v). R_{f-Start} = 0.0, R_{f-prod} = 0.6). After removal of the solvent, the residue was taken up into water (50 mL) and washed with CH₂Cl₂ (4 × 50 mL). The organic layer was dried over MgSO₄ (1 h) and the solvent removed under reduced pressure to give a yellow oil. Purification was achieved by column chromatography on silica gel (gradient elution from CH₂Cl₂ to CH₂Cl₂ : methanol, 95 : 5, v/v), to give the desired compound as a yellowish solid (0.619 g, 0.19, 63%). ¹H NMR (500 MHz, CDCl₃, 298 K): δ 6.00 (m, 7H; H8), 5.91 (m, 7H; H11), 5.27 (d, 7H; H9a), 5.24 (m, 7H; H1), 5.15 (d, 7H; H12a), 5.06 (d, 7H; H9b), 5.01 (d, 7H; H12b), 4.42 (dd, 7H; H7a), 4.22 (m, 7H; H7b), 4.19-4.12 (m, 14H; H10), 4.01 (br, 7H; H3), 3.74 (br d, 7H; H5), 3.63 (t, 7H; H4), 3.49 (d, 14H; H13a), 3.36 (d, 14H; H13b), 3.2 (m, 14H; H2, H6a), 3.13 (d, 7H; H6b), 1.44 (d, 126H; (*t*-Bu). ¹³C NMR (125 MHz, CDCl₃, 298 K): δ 170.9 (C14), 137.1 (C8), 135.8 (C11), 115.8 (C9), 114.9 (C12), 98.6 (C1), 80.2 (C15, C4), 79.6 (C3), 79.4 (C2), 74.2 (C7), 72.9 (C10), 71.5 (C5), 57.8 (C13), 55.1 (C6), 28.3 (t-Bu). FT-IR: 1733 cm⁻¹ (C=O stretching). During MALDI-TOF MS analysis the compound degraded.

**β-IDACYD-OH.** Heptakis[6-bis(*t*-butyloxycarbonylmethyl)amino-6-deoxy-2,3-di-*O*-allyl)-βCD (0.157 g, 0.048 mmol) was dissolved in CHCl₃ (1 mL) and it was stirred under Argon atmosphere. Trifluoroacetic acid (2.5 mL, 32.6 mmol) and *tetrakis* (triphenylphosphine)palladium(0) (0.327 g, 0.283 mmol) were added in different portions over the full reaction time (48 h) and the mixture was heated up at 80 °C. The catalyst was precipitated in neutralized water (NaOH 1M; final volume: 30 mL) and then filtered out after sonication and centrifugation. The solid residue was washed again with water (2 × 20 mL). The aqueous layers were combined and dried under *vacuum.* The crude product was re-dissolved in H₂O, the pH was adjusted to 7 (NaOH 1 M) and was dialyzed for 48 h. The solution was then filtered on paper and dried under *vacuum.* The residue was dissolved in H₂O (0.2 mL) and precipitated from acetone (2 × 2 mL). The solid was dried under *vacuum* to yield the desired compound (0.05 g, 50 %).¹H NMR (500 MHz, D₂O, pH 6, 298 K): δ 5.19 (s, 7H; H1), 4.25 (br, 7H; H5), 3.94 (br t, 7H; H3), 3.85 (br dd, 28H; H7), 3.69 (br t, 7H; H4), 3.61 (br, 14H; H2, H6a), 3.42 (br dd, 7H; 6Hb). ¹³C NMR (62.5 MHz, D₂O, pH 7, 298 K): δ 170.1 (C8), 99.9 (C1), 80.7 (C4), 71.8 (C3), 71.4 (C2), 67.0 (C5), 58.6 (C7), 55.7 (C6). FT-IR: 1615 cm⁻¹ (C=O stretching); 3240 cm⁻¹ (-OH stretching). MALDI-TOF MS: m/z: 1938 [M - H]⁻, 1960 [M - 2H + Na]⁻, 1982 [M - 3H + 2Na]⁻, 2004 [M - 4H + 3Na]⁻. Calc. mass [C₇₀H₁₀₅N₇O₅₆] = 1939.56.

Following the same general methods, the compounds according to Formula I where R is H and n is 1 (**α-IDACYD-OH**) can also be synthesized. Specifically, starting from the literature compound hexakis(6-azido-6-deoxy)-αCD (N. Mourtzis, K. Eliadou, C. Aggelidou, V. Sophianopoulou, I. M. Mavridis and K. Yannakopoulou, Per(6-guanidino-6-deoxy)cyclodextrins: synthesis, characterisation and binding behaviour toward selected small molecules and DNA, Org. Biomol. Chem., 2007, 5, 125-131) the following compounds are prepared in sequence: hexakis(6-azido-6-deoxy-2,3-di-*O*-allyl)-αCD, hexakis(6-amino-6-deoxy-2,3-di-*O*-allyl)-αCD, hexakis[6-bis(t-butyloxycarbonylmethyl)amino-6-deoxy-2,3-di-*O*-allyl)-αCD and **α**-**IDACYD-OH.** Likewise, following the same general methods, the compound according to Formula I where R is H and n is 3 **(γ-IDACYD-OH)** can also be synthesized. Specifically, starting from the literature compound octakis(6-azido-6-deoxy)-yCD (N. Mourtzis, K. Eliadou, C. Aggelidou, V. Sophianopoulou, I. M. Mavridis and K. Yannakopoulou, Per(6-guanidino-6-deoxy)cyclodextrins: synthesis, characterisation and binding behaviour toward selected small molecules and DNA, Org. Biomol. Chem., 2007, 5, 125-131) the following compounds are prepared in sequence: octakis(6-azido-6-deoxy-2,3-di-*O*-allyl)-γCD, octakis(6-amino-6-deoxy-2,3-di-*O*-allyl)-γCD, octakis[6-bis(t-butyloxycarbonylmethyl)amino-6-deoxy-2,3-di-*O*-allyl)-γCD and **γ-IDACYD-OH.**

***Example 3. In-vitro activity of IDA CYD compounds:*** The *in-vitro* inhibitory activity of **IDACYDs** against an MBL is demonstrated by NMR spectroscopy. As shown in Fig.2 [panel (a) and (b)] portions of the ¹H NMR spectra (500 MHz, 298K, phosphate buffer, H₂O (with a sealed capillary tube insert containing D₂O) of meropenem (0.18 mM) in the presence of an MBL containing crude enzyme preparation (Kpn LA-26 NDM-1, 485 µL), monitored over time: a) in the absence of **β-IDACYD** after 6 h incubation almost 100 % of the drug is hydrolyzed; b) in the presence of **β-IDACYD** (1.9 mM) no signals of hydrolysis of the drug could be observed over a 12 h period.

To verify the suggestive direct inhibitory effect on the MBLs we determined **β-IDACYD** IC₅₀ against NDM and VIM type MBLs spectrophotometrically. The inhibitor exhibited high potency against NDM-1 and VIM-1 enzymes: 1.626 µM ± 0.32 and 14.99 µM ± 1.07, respectively as shown in Fig.2 panel (c).

Further the *in-vitro* inhibitory activity of **IDACYDs** was tested against highly resistant pathogens. Tables 3, 4 and 5 show the effect of **IDACYDs** using a collection of 36 highly resistant Gram negative strains (Table 2, Kirby-Bauer disc diffusion method) producing NDM, VIM and IMP type MBLs as well as 6 isolates expressing serine beta-lactamases (ESBLs, AmpCs). It was determined that 50 µM was the minimal concentration exhibiting maximum inhibition against the MBL producing clinical strains, when tested in combination with ceftazidime, imipenem and meropenem. Specifically, in all cases 50 µM of the **β-IDACYD** reduced meropenem's and imipenem's minimum inhibitory concentration (MIC) up to 256-fold, re-sensitizing all MBL producing strains to carbapenems. Clinical isolates co-expressing ESBLs and/or AmpC β-lactamases along with MBLs where re-sensitized to both imipenem and meropenem, as expected.

**Table 2: Collection of clinical isolates used for in-vitro evaluation**

| **Bacterial species** | **Clinical isolate** | **β-lactamase** | **Phenotype** |
|---|---|---|---|
| ***Klebsiella pneumoniae*** | SEC-2 | VIM | tic^{r}, caz^{r}, amc^{r}, fep^{r}, tzp^{r}, fox^{r}, atm^{s} |
| | SEC-4 | VIM | tic^{r}, caz^{r}, amc^{r}, fep^{r}, tzp^{r}, fox^{r}, atm^{s} |
| | LA-109/16 | VIM, CTXM | tic^{r}, caz^{r}, amc^{r}, fep^{r}, tzp^{r}, fox^{r}, atmⁱ |
| | LA-82/16 | VIM, SHV | tic^{r}, caz^{r}, amc^{r}, fep^{r}, tzp^{r}, fox^{r}, atm^{r} |
| | LAR-2873 | VIM-12 | tic^{r}, caz^{r}, amc^{r}, fep^{r}, tzp^{r}, fox^{r}, atm^{r} |
| | ESDY-2681 | VIM-27 | tic^{r}, caz^{r}, amc^{r}, fep^{r}, tzp^{s}, fox^{r}, atm^{s} |
| | LA-34 | VIM, CMY | tic^{r}, caz^{r}, amc^{r}, fep^{r}, tzp^{r}, fox^{r}, atm^{r} |
| | ESDY-1780 | VIM, KPC | tic^{r}, caz^{r}, amc^{r}, fep^{r}, tzp^{r}, fox^{r}, atm^{r} |
| | 2685 | VIM, CMY, CTXM | tic^{r}, caz^{r}, amc^{r}, fep^{r}, tzp^{r}, fox^{r}, atm^{r} |
| | LA-26 | NDM | tic^{r}, caz^{r}, amc^{r}, fep^{r}, tzp^{r}, fox^{r}, atm^{r} |
| | 2489 | NDM | tic^{r}, caz^{r}, amc^{r}, fep^{r}, tzp^{r}, fox^{r}, atm^{r} |
| | LA-28 | NDM, CTXM | tic^{r}, caz^{r}, amc^{r}, fep^{r}, tzp^{r}, fox^{r}, atm^{r} |
| | LA-470/17 | NDM | tic^{r}, caz^{r}, amc^{r}, fep^{r}, tzp^{r}, fox^{r}, atm^{r} |
| | LA-419/16 | NDM,CTXM | tic^{r}, caz^{r}, amc^{r}, fep^{r}, tzp^{r}, fox^{r}, atm^{r} |
| | ESDY-5742 | IMP | tic^{r}, caz^{r}, amc^{r}, fep^{r}, tzp¹, fox^{r}, atm^{r} |
| | TZAN-59 | None | tic^{r}, caz^{s}, amc^{s}, fep^{s}, tzp^{s}, fox^{s}, atm^{s} |
| | 6/100 | VIM | tic^{r}, caz^{r}, amc^{r}, fep^{r}, tzp^{r}, fox^{r}, atm^{s} |
| | ALX-47 | OXA-48,CTX-M | tic^{r}, caz^{r}, amc^{r}, fep^{r}, tzp^{r}, fox^{s}, atm^{r} |
| | L078/11 | KPC | tic^{r}, caz^{r}, amc^{r}, fep^{r}, tzp^{r}, fox^{r}, atm^{r} |
| | LA-34 | VIM, CMY | tic^{r}, caz^{r}, amc^{r}, fep^{r}, tzp^{r}, fox^{r}, atm^{r} |
| | EY-20S | CMY, SHV | tic^{r}, caz^{r}, amc^{r}, fep^{r}, tzp¹, fox^{r}, atm^{r} |
| | IT-17829 | CTXM, SHV | tic^{r}, caz^{r}, amc^{r}, fepⁱ, tzp¹, fox^{r}, atm^{r} |
| | LA-38 | NDM | tic^{r}, caz^{r}, amc^{r}, fepⁱ, tzpⁱ, fox^{r}, atm^{s} |
| | LAR-40II/15 | KPC | tic^{r}, caz^{r}, amc^{r}, fep^{r}, tzp^{r}, fox^{r}, atm^{r} |
| | LAR-26I/15 | NDM, VIM, CTXM | tic^{r}, caz^{r}, amc^{r}, fep^{r}, tzp^{r}, fox^{r}, atm^{r} |
| | LAR-37II/15 | NDM, VIM, CTXM | tic^{r}, caz^{r}, amc^{r}, fep^{r}, tzp^{r}, fox^{r}, atm^{r} |
| | LAR-7547 | NDM, VIM, CTXM | tic^{r}, caz^{r}, amc^{r}, fep^{r}, tzp^{r}, fox^{r}, atm^{r} |
| | LAR-38I/15 | VIM, NDM, CTXM | tic^{r}, caz^{r}, amc^{r}, fep^{r}, tzp^{r}, fox^{r}, atm^{r} |
| | LA-27 | NDM, CTXM | tic^{r}, caz^{r}, amc^{r}, fep^{r}, tzp^{r}, fox^{r}, atm^{r} |
| | UoA 12/227 | VIM | tic^{r}, caz^{r}, amc^{r}, fep^{r}, tzp^{r}, fox^{r}, atm^{s} |
| | UoA 1995 | VIM | tic^{r}, caz^{r}, amc^{r}, fep^{r}, tzp^{r}, fox^{r}, atm^{s} |
| ***Proteus mirabilis*** | ALX-13 | VIM, VEB | tic^{r}, caz^{r}, amc^{r}, fep^{r}, tzp^{s}, fox^{s}, atm^{s} |
| | ESDY-17 | VIM, CMY | tic^{r}, caz^{r}, amc^{r}, fep^{r}, tzp^{s}, fox^{r}, atm^{s} |
| | ESDY-15315 | VIM, CMY | tic^{r}, caz^{r}, amc^{r}, fep^{r}, tzp^{r}, fox^{r}, atm^{s} |
| | ESDY-15184 | VIM, CMY | tic^{r}, caz^{r}, amc^{r}, fep^{r}, tzp^{s}, fox^{r}, atm^{s} |
| ***Providencia stuartii*** | ALX- 21 | VIM, VEB | tic^{r}, caz^{r}, amc^{r}, fep^{r}, tzp^{s}, fox^{r}, atm^{s} |
| | ALX-27 | VIM | tic^{r}, caz^{r}, amc^{r}, fep^{r}, tzp^{s}, fox^{s}, atmⁱ |
| | ESDY-4559 | VIM | tic^{r}, caz^{r}, amc^{r}, fep^{r}, tzp^{r}, foxⁱ, atm^{s} |
| | ESDY-4563 | VIM, VEB | tic^{r}, caz^{r}, amc^{r}, fep^{r}, tzp^{s}, fox^{s}, atmⁱ |
| ***Enterobacter gergoviae*** | AK-5227 | VIM, CMY | tic^{r}, caz^{r}, amc^{r}, fep^{r}, tzp^{r}, fox^{r}, atm^{r} |
| | AK-5240 | VIM, CMY | tic^{r}, caz^{r}, amc^{r}, fep^{r}, tzp^{s}, fox^{r}, atm^{s} |
| ***Acinetobacter baumannii*** | TZAN-40 | VIM, OXA-58 | gm^{r}, tm^{r}, an^{r}, net^{r}, cip^{r} |
| | TZAN-42 | VIM, OXA-58 | gm^{r}, tm^{r}, an^{r}, net^{r}, cip^{r} |
| ***Escherichia coli*** | TZAN-116 | VIM | tic^{r}, caz^{r}, amc^{r}, fep^{r}, tzp^{r}, fox^{r}, atm^{r} |
| | TZAN-541 | VIM | tic^{r}, caz^{r}, amc^{r}, fep^{r}, tzp^{r}, fox^{r}, atm^{r} |
| ***Serratia marcescens*** | EUG-815 | VIM, CMY | tic^{r}, caz^{r}, amc^{r}, fep^{r}, tzp^{r}, fox^{r}, atm^{s} |

| | | | |
|---|---|---|---|
| Tic; ticarcillin, caz; ceftazidime, amc; amoxicillin-clavulanic acid, fep; cefepime, tzp; piperacillin-tazobactam, fox; cefoxitin, atm; aztreonam. Symbol r stands for resistant whereas s stands for sensitive. Antibiotic susceptibility was determined using the Kirby-Bauer disc diffusion susceptibility testing. Strains were characterised based on the EUCAST clinical breakpoints v.07 (2017). | | | |

**Table 3: MIC values of carbapenems in the presence of β-IDACYD tested against resistant strains**

| **Bacterial species** | **Clinical isolate** | **Imipenem MIC(mg/L)** | **Imipenem /50µM β-IDACYD MIC(mg/L)** | **Meropenem MIC(mg/L)** | **Meropenem /50µM β-IDACYD MIC(mg/L)** |
|---|---|---|---|---|---|
| ***Klebsiella pneumoniae*** | SEC-2 | 32 | 2 | 64 | 2 |
| | SEC-4 | 64 | 2 | 32 | 1 |
| | LA-109/16 | 16 | <0.125 | 128 | <0.25 |
| | LA-82/16 | 8 | <0.125 | 64 | 0.25 |
| | LAR-2873 | 4 | <0.125 | 8 | <0.25 |
| | ESDY-2681 | 16 | <0.125 | 128 | <0.25 |
| | LA-34 | 32 | <0.5 | 256 | 0.25 |
| | ESDY-1780 | 16 | 8 | 64 | 32 |
| | 2685 | 32 | 0.5 | 128 | <0.25 |
| | LA-26 | 16 | <0.125 | 32 | <0.25 |
| | 2489 | 32 | 0.5 | 128 | 1 |
| | LA-28 | 8 | <0.125 | 64 | <0.25 |
| | LA-470/17 | 8 | 8 | 128 | 32 |
| | LA-419/16 | 4 | <0.125 | 16 | <0.25 |
| | ESDY-5742 | 8 | <0.125 | 16 | <0.25 |
| | TZAN-59 | 1 | 0.5 | 0.5 | 0.5 |
| | 6/100 | 32 | 1 | 128 | 1 |
| | ALX-47 | 4 | 4 | 8 | 4 |
| | L078/11 | 32 | 16 | 64 | 64 |
| | LA-34 | 32 | 0.25 | 64 | 0.25 |
| | EY-20S | 2 | 2 | 8 | 4 |
| | IT-17829 | 1 | 0.5 | 32 | 32 |
| | LA-38 | 8 | <0.125 | 128 | 0.5 |
| | LAR-40II/15 | 16 | 16 | 64 | 64 |
| | LAR-26I/15 | 16 | 0.125 | 64 | 0.5 |
| | LAR-37II/15 | 64 | <0.125 | 128 | <0.25 |
| | LAR-7547 | 64 | 4 | 128 | 16 |
| | LAR-38I/15 | 4 | <0.125 | 16 | <0.25 |
| | LA-27 | 8 | <0.125 | 8 | <0.25 |
| | UoA 12/227 | 8 | <0.125 | 64 | <0.25 |
| | UoA 1995 | 8 | <0.125 | 64 | <0.25 |
| ***Proteus mirabilis*** | ALX-13 | 8 | 1 | 32 | 0.5 |
| | ESDY-17 | 4 | <0.125 | 0.25 | <0.25 |
| | ESDY-15315 | 16 | <0.125 | 4 | <0.25 |
| | ESDY-15184 | 8 | 0.125 | 2 | <0.25 |
| ***Providencia stuartii*** | ALX- 21 | 8 | 0.25 | 2 | <0.25 |
| | ALX-27 | 16 | <0.125 | 2 | <0.25 |
| | ESDY-4559 | 4 | <0.125 | 4 | <0.25 |
| | ESDY-4563 | 4 | <0.125 | 2 | <0.25 |
| ***Enterobacter gergoviae*** | AK-5227 | 8 | 1 | 2 | <0.25 |
| | AK-5240 | 8 | <0.125 | 1 | <0.25 |
| ***Acinetobacter baumannii*** | TZAN-40 | 4 | 0.25 | 4 | 2 |
| | TZAN-42 | 8 | 2 | 32 | 8 |
| ***Escherichia coli*** | TZAN-116 | 4 | <0.125 | 1 | <0.25 |
| | TZAN-541 | 4 | <0.125 | 2 | <0.25 |
| ***Serratia marcescens*** | EUG-815 | 16 | <0.125 | 256 | <0.25 |

**Table 4: MIC values of 3^{rd} generation cephalosporin (ceftazidime) in the presence of β-IDACYD tested against resistant strains**

| **Bacterial species** | **Clinical isolate** | **Ceftazidime MIC(mg/L)** | **Ceftazidime/ 50µM β-IDACYD MIC(mg/L)** |
|---|---|---|---|
| ***Klebsiella pneumoniae*** | LA-109/16 | >256 | 32 |
| | LA-82/16 | >256 | 32 |
| | LA-28 | >256 | 32 |
| | ESDY-5742 | >256 | 1 |
| | ALX-47 | >256 | >256 |
| | LAR-4011/15 | 32 | 32 |
| | UoA 12/227 | >256 | <0.25 |
| | UoA 1995 | >256 | <0.25 |
| ***Proteus mirabilis*** | ESDY-15184 | >256 | <0.25 |
| ***Providencia stuartii*** | ALX- 21 | 128 | 16 |
| | ESDY-4559 | 8 | <0.25 |
| ***Escherichia coli*** | TZAN-116 | >256 | <0.25 |
| ***Serratia marcescens*** | EUG-815 | >256 | <0.25 |

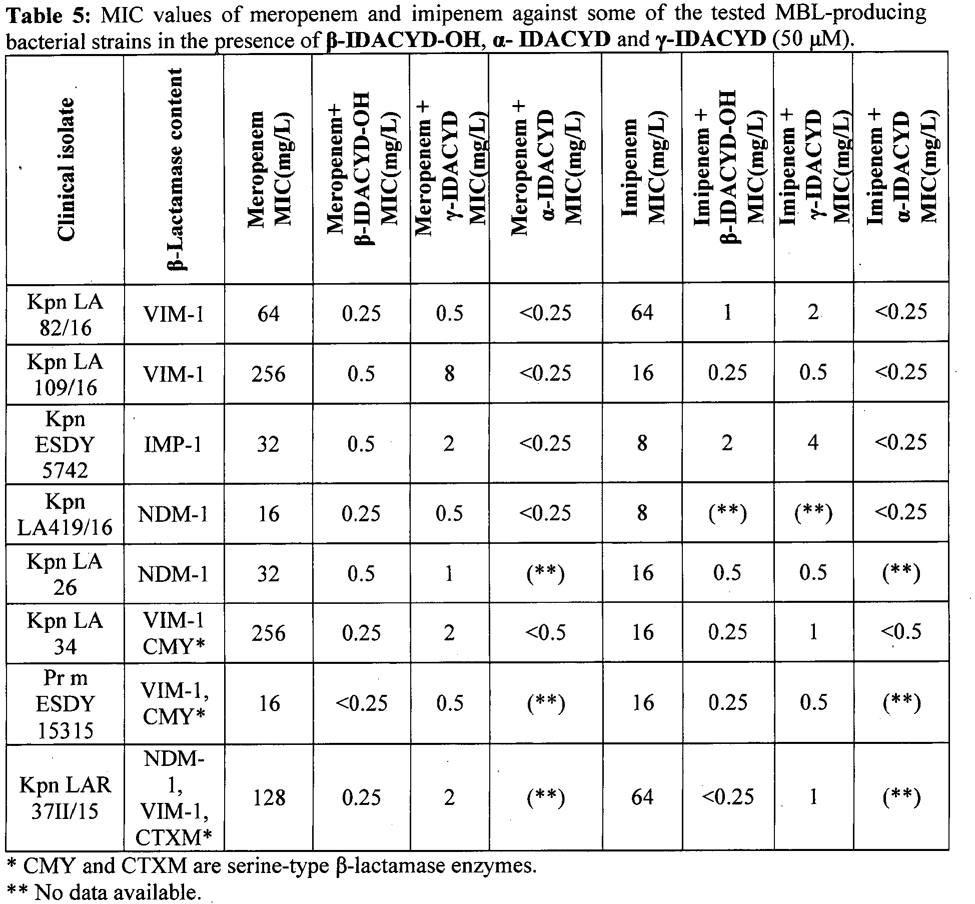

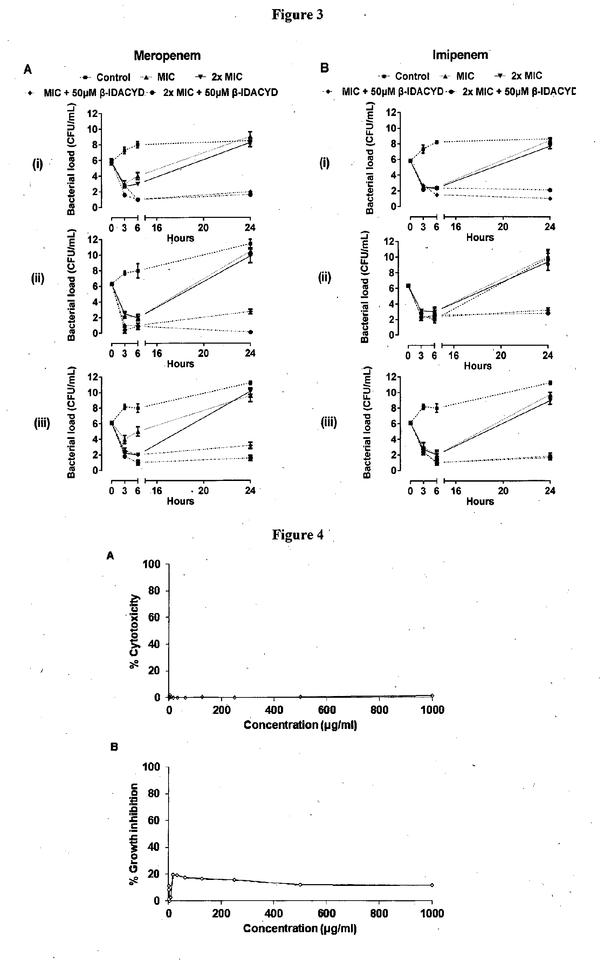

Time kill experiments demonstrated the *in-vitro* potency of **β-IDACYD**. The assays were performed for imipenem and meropenem using concentrations corresponding to the MIC and the 2xMIC values against NDM, VIM and IMP producing *K. pneumoniae* clinical isolates of standard bacterial inoculum (10⁵-10⁶ CFU/mL). In Fig. 3 the results of the time-kill assays are shown. **(i)** NDM-1 producing *K. pneumoniae,* clinical strain LA26, **(ii)** VIM-1 producing *K. pneumoniae* clinical strain LA 109/16 and (**ii**i) IMP producing *K. pneumoniae* clinical strain 5742, using meropenem (**A**) and imipenem (**B**) at inhibitory concentrations equal to the MIC and 2xMIC in the presence or absence of 50 µM **β-IDACYD**. Results are expressed as log₁₀ CFU/mL. Experiments were performed in duplicate. Antibiotic-free control cultures were included in each set of experiments as controls. Error bars are indicative of standard error mean (SEM) (n=2 replicates). In all cases the bacterial load remains close to zero only if **β-IDACYD** is added to MIC or 2xMIC concentration solutions of meropenem (upper panels) or imipenem (lower panels). In all cases, at the end of a 24 hour incubation the presence of 50 µM **β-IDACYD** resulted in a >8 log₁₀ reduction of the bacterial load (CFU/mL) compared to that of the antibiotic alone.

***Example 4. In-vitro and in-vivo toxicity experiments:*** There is no *in-vitro* cytotoxicity of **β-IDACYD** as shown in Fig. 4 at concentrations ranging from 0.5 to 1,000 mg/L using CytoTox 96 Non-Radioactive Cytotoxicity Assay (A) and CellTiter 96 Aqueous One Solution Cell Proliferation Assay (B) for L929 fibroblast cells treated with different concentration of **β-IDACYD** for 24 h. Results are presented as means of two replicates. The cells did not present any morphological differences characteristic of cytotoxicity when examined under a microscope.

The acute *in-vivo* toxicity of **β-IDACYD** was also determined to provide range-finding doses to be used in *in-vivo* studies and possibly implicate target organs for toxicity. For dosing schemes up to 500 mg/kg there were no deaths and no signs indicative of acute toxicity.

***Example 5. Stability and Biodistribution of β-IDACYD:*** Radiolabelling with Gallium-67 ion (⁶⁷Ga) using a large excess (4 × 10⁴-fold) of **β-IDACYD** was performed resulting in a single radioactive species with high radiochemical purity (> 95% by radio-HPLC assessment). The stability of radiolabelled **⁶⁷Ga-β-IDACYD** was assessed in the presence of excess of apo-transferrin. The radiotracer was fully intact after 120 min, with no HPLC evidence of ⁶⁷Ga-apotransferrin binding or loss of ⁶⁷Ga, thus suggesting high *in-vitro* stability.

Metabolite studies were performed on blood and urine samples taken from CD-1 mice injected intravenously with 10 mg/kg **⁶⁷Ga-β-IDACYD**, at 5, 60 and 120 min post-injection. As demonstrated by radio-HPLC analysis, a single peak corresponding to intact **⁶⁷Ga-β-IDACYD** was observed in all evaluated samples, with no other radioactive components present, suggesting very high *in vivo* metabolic stability of **⁶⁷Ga-β-IDACYD.**

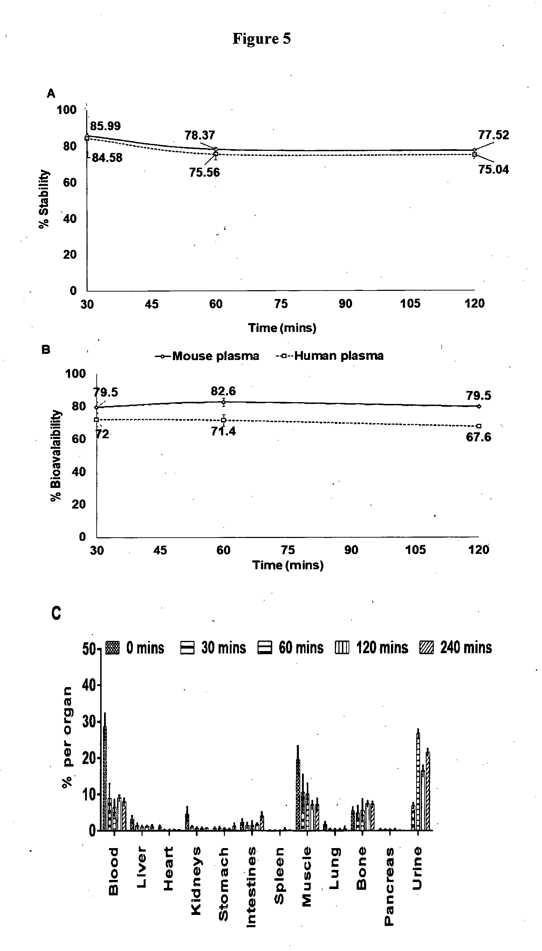

Incubation of **⁶⁷Ga-β-IDACYD** in fresh human and mouse plasma at 37 °C also showed high stability at all time-points assessed (Fig. 5A), with **⁶⁷Ga-β-IDACYD** demonstrating slightly higher plasma stability in mouse than in human plasma (78% intact **⁶⁷Ga-β-IDACYD** after 120 min incubation, compared to 72% intact **⁶⁷Ga-β-IDACYD** in human plasma). A fraction of approximately 30% of **⁶⁷Ga-β-IDACYD** was found to be bound to human plasma proteins at 30 min post-injection (p.i.), remaining stable up to 120 min p.i. Binding of **⁶⁷Ga-β-IDACYD** to mouse plasma proteins was almost 20% at 120 min demonstrating high bioavailability (Fig. 5B).

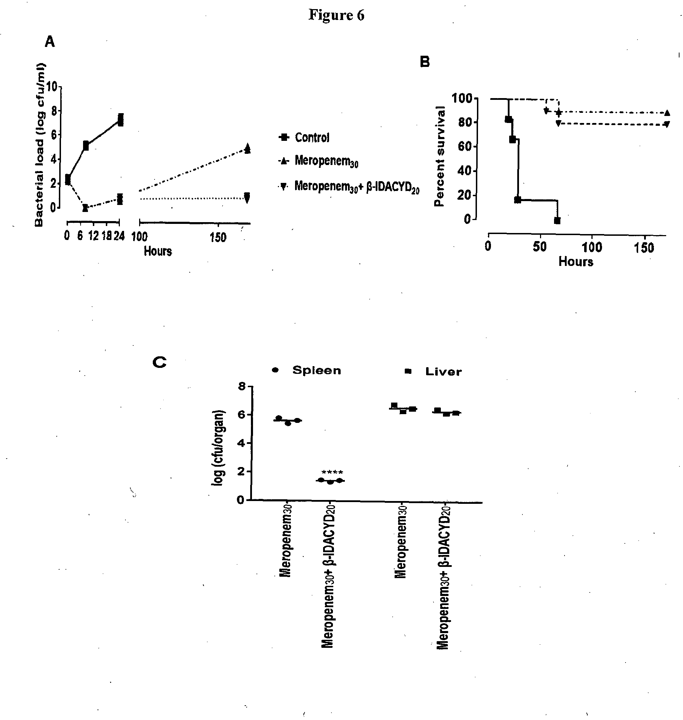

Biodistribution experiments on CD-1 mice using the radiolabelled **⁶⁷Ga:β-IDACYD** complex was evaluated at 5, 30, 60, 120 and 240 min (n=10/time point) post-injection of the radiotracer (Fig. 6c). The excretion route of the radiotracer was primarily *via* the urinary tract and no significant liver, spleen or intestinal uptake or accumulation was observed. Significant amounts of the **⁶⁷Ga:β-IDACYD** complex remained in the blood and in muscle tissue (Fig. 5C).

Since the *in-vitro* potency of **β-IDACYD** was remarkable and its *in-vivo* toxicity profile safe, an initial *in-vivo* localised infection model was performed to exploit its *in-vivo* therapeutic capabilities.

***Example 6. In-vivo murine thigh infection model:*** A murine thigh infection model was utilized based on the biodistribution results. In a neutropenic mouse thigh infection model was administered intravenously a therapeutic scheme of either 30 mg/kg imipenem or 30 mg/kg imipenem co-administered with 10 mg/kg **β-IDACYD** in 2 h intervals for 24 hours. At the end of the treatment the bacterial burden of the thigh was reduced by 0.5 log₁₀ in the presence of 10 mg/kg **β-IDACYD** compared to that of the antibiotic monotherapy.

Twenty, 10 weeks old female (CD-1) mice were rendered neutropenic by administration of two doses of cyclophosphamide (150 mg/kg and 100 mg/kg per mouse on days -4 and -1, respectively). On day 0, all mice were challenged with 0.1 mL of a 10⁷ CFU/mL of VIM-1 producing *K*. *pneumoniae* SEC-4 by intramuscular injection in the right thigh. From the six mice in the control group that were treated with normal saline (Group A), two mice were sacrificed 2 hours post inoculation while the other mice in groups of seven mice each were intravenously treated with 30 mg/kg imipenem (Group B) or 30 mg/kg imipenem along with 10 mg/kg **β-IDACYD** (Group C) at 2 hour intervals for 24 hours. At 6-hours post inoculation two mice of group 1 as well as three mice of each of groups 2 and 3 were sacrificed and their thighs were aseptically removed. At 24-hours the remaining mice from all groups were sacrificed and their thighs were removed as before. The thigh muscles were homogenized in 1 mL of sterile normal saline with a manual homogenizer. Serial ten-fold dilutions of the thigh homogenates were prepared in sterile normal saline and 0.1 mL of the dilutions for each thigh was plated onto Mueller-Hinton agar plates, in duplicate. Bacterial colonies were enumerated for each plate following ~16 hours of incubation at 37° C. At the end of the treatment the bacterial burden of the thigh was reduced by 0.5log₁₀ in the presence of 10 mg/kg **β-IDACYD** compared to that of the antibiotic monotherapy.

Since the results of the initial experiments were promising, *in-vivo* experiments were performed using a systemic infection model (sepsis) for assessing the therapeutic efficacy of the combinational therapies. A sepsis model was deemed suitable for *in-vivo* therapeutic testing since the biodistribution studies showed a considerable concentration of **β-IDACYD** in the blood within a 2h period. A set of three *in-vivo* experiments were performed.

***Example 7. In-vivo murine sepsis model: 2-hour intervals treatment scheme:*** Infection was induced intraperitoneally in neutropenic 7-8 weeks old, female CD-1 mice, weighing 28-32 g by injection of 0.1 mL of bacterial suspension of the NDM producing *K. pneumoniae* clinical strain LA26 (10⁸ CFU/mL) (imipenem MIC 16 mg/L) derived from log-phase cultures grown in LB broth. The animals were rendered neutropenic (<100 neutrophils/mm³) by intraperitoneal injection of 150 and 100 mg/kg of cyclophosphamide at day -4 and day -1, respectively, before infection. Treatment with meropenem in presence or absence of **β-IDACYD** was started 2 h after inoculation and lasted for 24 h. The meropenem/ **β-IDACYD** combination was administrated in all dosing schemes simultaneously. Three dosing schemes were used: (A) 30 mg/kg meropenem, (B) 30 mg/kg meropenem with 20 mg/kg **β-IDACYD** and (C) a control group that received normal saline every 2 h by intraperitoneal injection (total daily doses of 360 mg/kg meropenem (A) and 360 mg/kg meropenem with 240 mg/kg **β-IDACYD** (B)). Blood samples were taken 0, 6, 24 hours and 7 days after injection of a single dose of antibiotic (five infected animals for each timepoint) were serially diluted in normal saline (10⁻¹ to 10⁻⁹) and plated on MH agar plates which were incubated for 16 hours at 37°C for determination of viable organisms. All mice that were alive were euthanised 7 days post infection and spleen and liver were harvested. The organs were placed into 1 mL of normal saline on ice and then homogenised. The organ homogenates were then serially diluted in normal saline (10⁻¹ to 10⁻⁹) and plated on MH agar for CFU enumeration. Without therapy 90% of mice succumbed to the infection within 24 hours. Meropenem monotherapy protected 80% of the mice for the duration of the experiment (140 hours) whereas the meropenem/**β-IDACYD** co-therapy protected 90% of mice for the same duration. Furthermore, at 140 hours post treatment initiation the meropenem/**β-IDACYD** combinatorial scheme resulted in the reduction of the bacterial load in blood by 4.0log₁₀ compared to that of the meropenem monotherapy. Finally, the bacterial load in the spleen was relieved by 4.2 log₁₀ compared to that of the antibiotic itself. In Fig.6 is shown the bacterial load (CFU/mL) (A) and survival percentage (B) of CD-1 mice infected with a lethal dose (10⁷ CFU) of NDM-1 producing *K. pneumoniae* strain LA26. Mice received intraperitoneally the following treatment scheme: normal saline, 30 mg/kg meropenem, 30 mg/kg meropenem+20mg/kg **β-IDACYD.** Treatment (initiated at 2 hours post infection) was given every 2h for a 24h period and blood was drawn 0 h, 6 h, 24 h and 140 h post treatment initiation. All groups were of n=10. Spleen and liver (C) from n=5 mice were also removed at 140 h post-treatment initiation the bacterial load [log (CFU/organ)] was determined. Error bars are indicative of SEM (n=2 replicates).

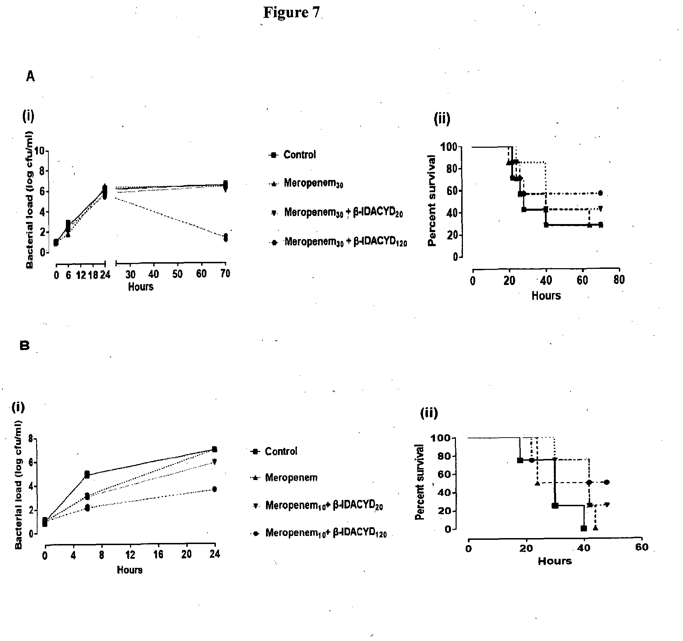

***Example 8. In-vivo murine sepsis model: Single dose treatment scheme-1:*** Neutropenic 7-8 weeks old CD-1 female mice weighing 29 to 32 grams were infected interperitoneally with a lethal dose (10⁷ CFU) of the NDM-1 producing *K. pneumoniae* clinical strain LA 26 as described previously. Treatment with meropenem in the presence or absence of **β-IDACYD** was given 2 h after the infection was established in a form of a single dose of (A) 30 mg/kg meropenem (n=10) or (B) 30 mg/kg meropenem with 20 mg/kg **β-IDACYD**(n=10) or (C) 30 mg/kg meropenem with 120 mg/kg **β-IDACYD**(n=10) and (D) a control group that received normal saline (n=10). The meropenem/ **β-IDACYD** combination was administrated in all dosing schemes simultaneously. Blood samples were taken 0, 6, 24 hours and 3 days after injection of a single dose of antibiotic (five infected animals for each timepoint) and were serially diluted in normal saline (10⁻¹ to 10⁻⁹) and plated on MH agar plates which were incubated for 16 hours at 37 °C for determination of viable organisms. All mice were euthanised 3 days post infection and spleen and liver were harvested. Organs were placed into 1 mL of normal saline on ice and then homogenised. Organ homogenates were then serially diluted in normal saline (10⁻¹ to 10⁻⁹) and plated on MH agar plates for CFU enumeration. In the absence of therapy 30% of mice remained alive 70 hours post infection. The same percentage of survival was observed for the meropenem monotherapy whereas a single dose of 120. mg/kg of **β-IDACYD** rescued 60% of mice at the same timeframe. The bacterial burden in the blood 70 hours post treatment was relieved by 4.8log₁₀ in the presence of 120 mg/kg of **β-IDACYD** compared to that of the antibiotic monotherapy. The bacterial load was significantly less both in the spleen (3.8log₁₀ reduction) and the liver (1.4log₁₀ reduction). In Fig. 7 is shown the bacterial load (CFU/mL) and survival percentage of CD-1 mice infected with a lethal dose (10⁷ CFU) of NDM-1 producing *K. pneumoniae* strain LA26. Mice received intraperitoneally one of the following treatment schemes: (panel A) normal saline, meropenem 30 mg/kg, meropenem, 30 mg/kg meropenem+20 mg/kg **β-IDACYD** or 30 mg/kg meropenem+120 mg/kg **β-IDACYD.** Treatment was given in a form of a single shot 2h after infection and blood was drawn 0h, 6h, 24h and 70h post treatment initiation. All groups were of n=10. Error bars are indicative of SEM (n=2 replicates).

***Example 9. In-vivo murine sepsis treatment model: Single dose treatment scheme-2:*** Infected, neutropenic mice received a single administration of (A) meropenem (10 mg/kg) or (B) meropenem (10 mg/kg) and **β-IDACYD** (20 mg/kg) or (C) meropenem (10 mg/kg) and **β-IDACYD** (120 mg/kg) or (D) normal saline (control) as described previously. Blood samples were collected at 0, 6 and 24 hours after injection of a single dose of antibiotic (4 infected animals for each timepoint), were serially diluted in normal saline (10⁻¹ to 10⁻⁹) and plated on MH agar plates which were incubated for 16 hours at 37°C for determination of viable organisms. The meropenem monotherapy was unable to protect any mice from death within 48 h whereas the same dose of meropenem co-administered with 120 mg/kg **β-IDACYD** rescued 50% of the mice within the same time. The resulting 3.0log₁₀ reduction in the blood bacterial load in the presence of 120 mg/kg of the inhibitor **β-IDACYD** compared to the antibiotic itself confirmed the high repotentiation abilities of the inhibitor to carbapenems even for suboptimal antibiotic doses. In Fig. 7 shows the bacterial load (CFU/mL) and survival percentage of CD-1 mice infected with a lethal dose (10⁷ CFU) of NDM-1 producing *K. pneumoniae* strain LA26. Mice received intraperitoneally one of the following treatment schemes: (panel B) normal saline, meropenem 10 mg/kg, meropenem, 10 mg/kg meropenem+20 mg/kg **β-IDACYD** or 10 mg/kg meropenem+120 mg/kg **β-IDACYD.** Treatment was given in a form of a single shot 2h after infection and blood was drawn 0h, 6h and 24h post treatment initiation. All groups were of n=10. Error bars are indicative of SEM (n=2 replicates).

In all cases, **β-IDACYD** presented long term suppression abilities of the bacteraemia induced in CD-1 mice as well as of the bacterial burden present in the spleen and liver.

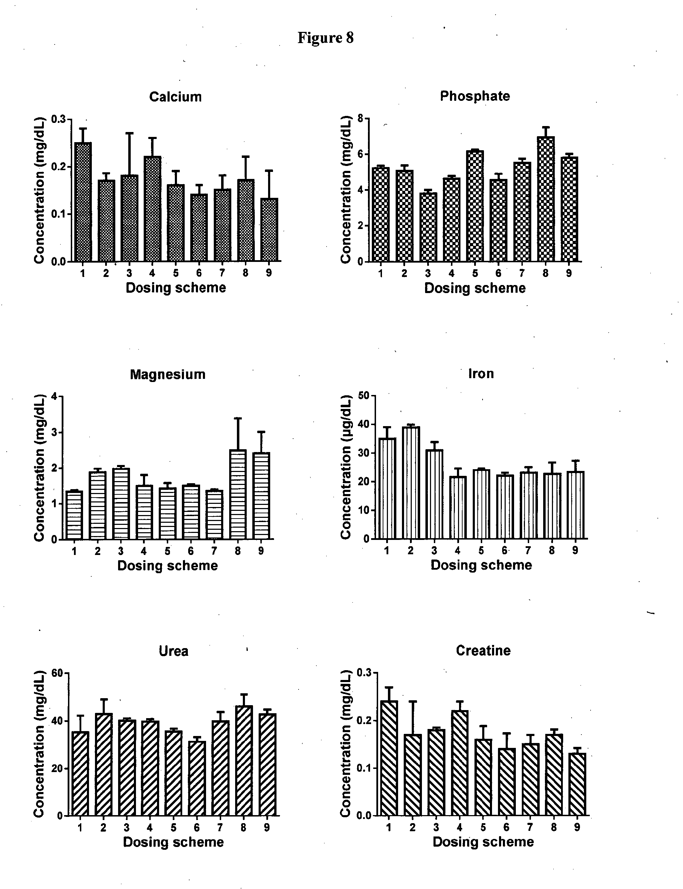

***Example 10. Examination of the cross-reactivity of* β-IDACYD *with vital metal ions in mouse plasma and of induction of immune responses in infected mice:*** In order to examine a potential cross-reactivity of the **β-IDACYD** with metal ions necessary for the maintenance of homeostasis, plasma was isolated by centrifugation (3,000 rpm, 5 min) from all blood samples collected. The plasma levels of four metal ions (calcium, magnesium, phosphate and iron) as well as the levels of creatinine and urea were determined using an Olympus AU 600 analyser. All ionic and organic compound levels were well within the control group range at all times indicating no disruption of the ionic balance and no signs of kidney function impairment (Figure 8). In Fig. 8 is shown the plasma concentration in mg/dL of calcium, iron, phosphate and magnesium ions, as well as urea and creatinine derived from CD-1 mice infected with a lethal dose (10⁷ CFU) of NDM-1 producing *K. pneumoniae* strain LA26. Mice received intraperitoneally normal saline, 30 mg/kg meropenem, 30 mg/kg meropenem+20mg/kg **β-IDACYD** combination administrated simultaneously. Treatment was given every 2h for a 24h period and blood was drawn and plasma was isolated at time points 0h, 6h, 24h and 140h (7d) post treatment initiation. The dosing scheme numbers correspond to the following conditions: (1) control at 0h, (2) control at 6h, (3) meropenem 30mg/kg at 6h, (4) meropenem 30mg/kg +20mg/kg **β-IDACYD** at 6h, (5) control at 24h, (6) meropenem 30mg/kg at 24 h, (7) meropenem 30mg/kg +20mg/kg **β-IDACYD**, (8) meropenem 30mg/kg at 140 h, (9) meropenem 30mg/kg +20mg/kg β-IDACYD at 140h.

As a way to further measure and evaluate the *in-vivo* potency and safety of **β-IDACYD**, we determined the levels of a series of lymphokines, cytokines, and pro-inflammatory indicator proteins. The levels of Th17 immune response related ILs 17E, 6, 1β, 4, 21, 10, 17A, 17F, IFN-g, MIP-3a, TNF-a and TNF-b were all determined in a luminex assay using plasma isolated from the mice involved in all the sepsis therapeutic schemes. The presence of **β-IDACYD** at no times induced an immune response, following the same immune response pattern as the antibiotic monotherapy. The observation was supported by the levels of the inflammatory response proteins CRP and SAP.

## Claims

1. A compound of Formula I 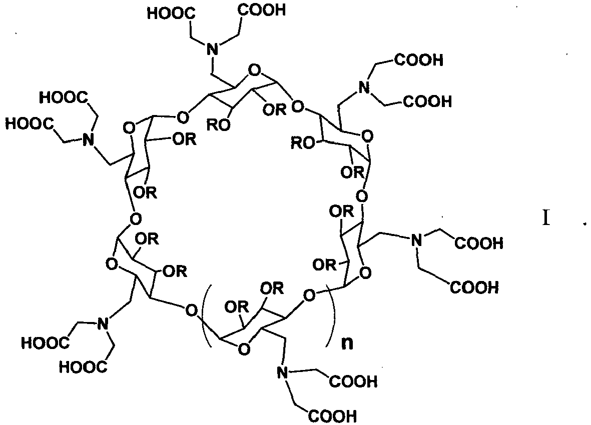 wherein R is CH₃ or H, and n is 1, 2 or 3, for use in the treatment of a bacterial infection caused by a Gram-negative bacterium producing a metallo-p-lactamase, wherein the compound is administered in combination with a β-lactam antimicrobial agent.

2. A compound for use according to claim 1, wherein R is CH₃ and n is 2.

3. A compound for use according to claim 1 or 2, wherein the β-lactam antimicrobial agent is a cephalosporin or a carbapenem.

4. A compound for use according to claim 3, wherein the β-lactam antimicrobial agent is meropenem or imipenem.

5. A compound for use according to any one of the preceding claims, wherein the bacterial infection is selected from blood stream infection, septicemia, sepsis, septic shock, upper respiratory tract infection, lower respiratory tract infection, lung abscess, urinary tract infection, intra-abdominal infection, surgical wound infection, endocarditis, skin and soft tissue infection, thrombophlevitis, osteomyelitis, septic arthritis, endophthalmitis, cystic fibrosis lung infection, brain abscess.

6. A compound for use according to any one of the preceding claims, wherein the compound is administered at a dosage of 5 to 500 mg/kg.

7. A compound for use according to claim 6, wherein the compound is administered at a dosage of 10 to 120 mg/kg.

8. A compound for use according to any one of the preceding claims, wherein the ratio between the administered amount of the β-lactam antimicrobial agent and the compound according to Formula I is from 1/0.3 to 1/15.

9. A compound for use according to claim 8, wherein the ratio between the administered amount of the β-lactam antimicrobial agent and the compound according to Formula I is from 1/0.7 to 1/12.

10. A compound for use according to any one of the preceding claims, wherein the compound is administered via the oral or the parenteral route.

11. A compound for use according to any one of the preceding claims, wherein the compound and the β-lactam antimicrobial agent are administered simultaneously or successively.

12. A pharmaceutical composition comprising a compound according to Formula I for use in the treatment of a bacterial infection according to any one of claims 1 to 10.

13. A pharmaceutical composition for use according to claim 11, wherein the composition further comprises a β-lactam antimicrobial agent.

14. A pharmaceutical composition for use according to claim 13, wherein the β-lactam antimicrobial agent is a cephalosporin or a carbapenem, preferably meropenem or imipenem.

15. A compound according to Formula I, according to claim 1, 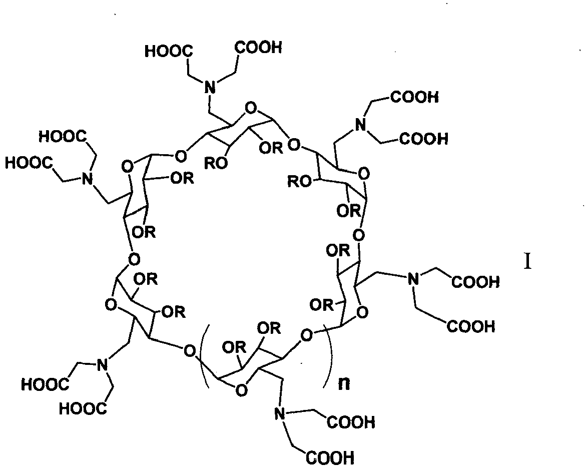 wherein R is H and n is 1, 2 or 3, in combination with a β-lactam antimicrobial agent.

## Patentansprüche

1. Verbindung der Formel I 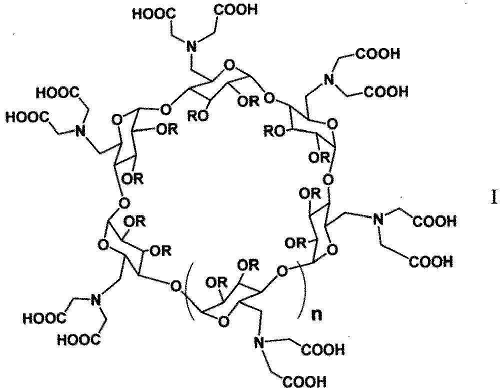 wobei R CH₃ oder H ist und n 1, 2 oder 3 beträgt, zur Verwendung bei der Behandlung einer Bakterieninfektion, die durch eine gramnegative Bakterie verursacht wird, die eine Metallo-β-lactamase erzeugt, wobei die Verbindung in Kombination mit einem antimikrobiellen β-Lactamwirkstoff verabreicht wird.

2. Verbindung zur Verwendung nach Anspruch 1, wobei R CH₃ ist und n 2 beträgt.

3. Verbindung zur Verwendung nach Anspruch 1 oder 2, wobei der antimikrobielle β-Lactamwirkstoff ein Cephalosporin oder ein Carbapenem ist.

4. Verbindung zur Verwendung nach Anspruch 3, wobei der antimikrobielle ß-Lactamwirkstoff Meropenem oder Imipenem ist.

5. Verbindung zur Verwendung nach einem der vorhergehenden Ansprüche, wobei die Bakterieninfektion ausgewählt ist unter Blutstrominfektion, Septikämie, Sepsis, septischem Schock, Infektion der oberen Atemwege, Infektion der unteren Atemwege, Lungenabszess, Harntraktinfektion, intraabdomineller Infektion, Operationswundeninfektion, Endokarditis, Haut- und Weichgewebeinfektion, Thrombophlebitis, Osteomyelitis, septischer Arthritis, Endophthalmitis, zystische Fibrose, Lungeninfektion, Gehirnabszess.

6. Verbindung zur Verwendung nach einem der vorhergehenden Ansprüche, wobei die Verbindung in einer Dosis von 5 bis 500 mg/kg verabreicht wird.

7. Verbindung zur Verwendung nach Anspruch 6, wobei die Verbindung in einer Dosis von 10 bis 120 mg/kg verabreicht wird.

8. Verbindung zur Verwendung nach einem der vorhergehenden Ansprüche, wobei das Verhältnis zwischen der verabreichten Menge des antimikrobiellen ß-Lactamwirkstoffs und der Verbindung der Formel I entsprechend 1/0,3 bis 1/15 beträgt.

9. Verbindung zur Verwendung nach Anspruch 8, wobei das Verhältnis zwischen der verabreichten Menge des antimikrobiellen β-Lactamwirkstoffs und der Verbindung der Formel I entsprechend 1/0,7 bis 1/12 beträgt.

10. Verbindung zur Verwendung nach einem der vorhergehenden Ansprüche, wobei die Verbindung auf oralem oder parentheralem Weg verabreicht wird.

11. Verbindung zur Verwendung nach einem der vorhergehenden Ansprüche, wobei die Verbindung und der antimikrobielle β-Lactamwirkstoff gleichzeitig oder nacheinander verabreicht werden.

12. Pharmazeutische Zusammensetzung umfassend eine Verbindung der Formel I entsprechend zur Verwendung bei der Behandlung einer Bakterieninfektion nach einem der Ansprüche 1 bis 10.

13. Pharmazeutische Zusammensetzung zur Verwendung nach Anspruch 11, wobei die Zusammensetzung ferner einen antimikrobiellen β-Lactamwirkstoff umfasst.

14. Pharmazeutische Zusammensetzung zur Verwendung nach Anspruch 13, wobei der antimikrobielle β-Lactamwirkstoff ein Cephalosporin oder ein Carbapenem, bevorzugt Meropenem oder Imipenem ist.

15. Verbindung der Formel I entsprechend nach Anspruch 1, 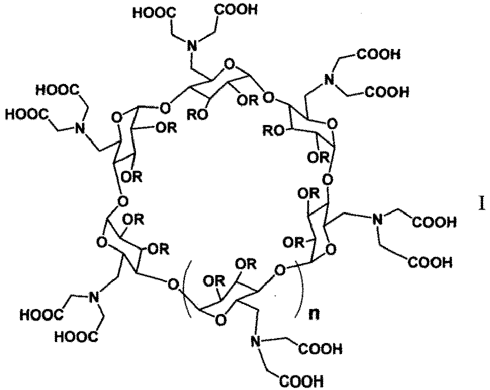 wobei R H ist und n 1, 2 oder 3 beträgt, in Kombination mit einem antimikrobiellen β-Lactamwirkstoff.

## Revendications

1. Composé de formule I 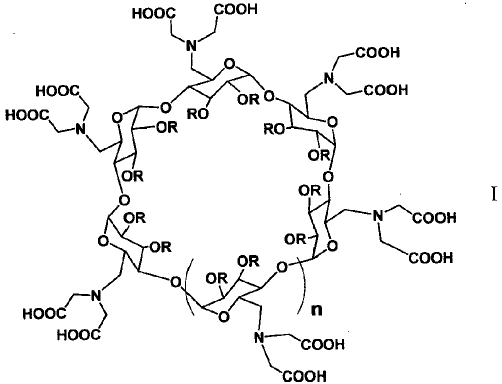 dans laquelle R est un groupe CH₃ ou H et n vaut 1, 2 ou 3, destiné à être utilisé dans le traitement d'une infection bactérienne provoquée par une bactérie Gram-négative produisant une métallo-β-lactamase, le composé étant administré en combinaison avec un agent antimicrobien β-lactame.

2. Composé destiné à être utilisé selon la revendication 1, dans lequel R est un groupe CH₃ et n vaut 2.

3. Composé destiné à être utilisé selon la revendication 1 ou 2, dans lequel l'agent antimicrobien β-lactame est une céphalosporine ou un carbapénem.

4. Composé destiné à être utilisé selon la revendication 3, dans lequel l'agent antimicrobien β-lactame est le méropénem ou l'imipénem.

5. Composé destiné à être utilisé selon l'une quelconque des revendications précédentes, dans lequel l'infection bactérienne est choisie parmi l'infection du flot sanguin, la septicémie, la sepsie, le choc septique, l'infection du système respiratoire supérieur, infection du système respiratoire inférieur, un abcès du poumon, l'infection du tractus urinaire, l'infection intra-abdominale, l'infection de blessure chirurgicale, l'endocardite, l'infection de la peau et du tissu mou, la thrombophlévite, l'ostéomyélite, l'arthrite septique, l'endophtalmite, l'infection pulmonaire de fibrose cystique, un abcès du cerveau.

6. Composé destiné à être utilisé selon l'une quelconque des revendications précédentes, dans lequel le composé est administré à une dose de 5 à 500 mg/kg.

7. Composé destiné à être utilisé selon la revendication 6, dans lequel le composé est administré à une dose de 10 à 120 mg/kg.

8. Composé destiné à être utilisé selon l'une quelconque des revendications précédentes, dans lequel le rapport entre la quantité administrée de l'agent antimicrobien β-lactame et le composé selon la formule I est de 1/0,3 à 1/15.

9. Composé destiné à être utilisé selon la revendication 8, dans lequel le rapport entre la quantité administrée de l'agent antimicrobien β-lactame et le composé selon la formule I est de 1/0,7 à 1/12.

10. Composé destiné à être utilisé selon l'une quelconque des revendications précédentes, dans lequel le composé est administré via la voie orale ou parentérale.

11. Composé destiné à être utilisé selon l'une quelconque des revendications précédentes, dans lequel le composé et l'agent antimicrobien β-lactame sont administrés simultanément ou successivement.

12. Composition pharmaceutique comprenant un composé selon la formule I destiné à être utilisé dans le traitement d'une infection bactérienne selon l'une quelconque des revendications 1 à 10.

13. Composition pharmaceutique destinée à être utilisée selon la revendication 11, dans laquelle la composition comprend en outre un agent antimicrobien β-lactame.

14. Composition pharmaceutique destinée à être utilisée selon la revendication 13, dans laquelle l'agent antimicrobien β-lactame est une céphalosporine ou un carbapénem, de préférence le méropénem ou l'imipénem.

15. Composé selon la formule I, selon la revendication 1, 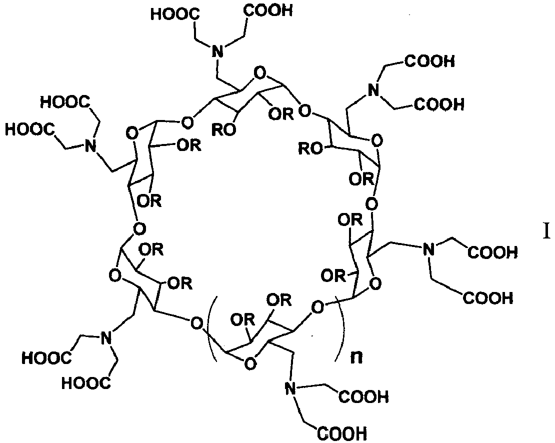 dans laquelle R est H et n vaut 1, 2 ou 3, en combinaison avec un agent antimicrobien β-lactame.
